# EUROPEAN PATENT SPECIFICATION

(11) **EP 1 877 061 B1**
(45) Date of publication and mention of the grant of the patent: **13.06.2012**
(21) Application number: 06758431.8
(22) Date of filing: 18.04.2006
(51) Int. Cl.: A61K 31/56

(54) **METHOD AND COMPOSITION FOR INHIBITING CELL PROLIFERATION AND ANGIOGENESIS**
VERFAHREN UND ZUSAMMENSETZUNG ZUR HEMMUNG VON ZELLPROLIFERATION UND ANGIOGENESE
METHODE ET COMPOSITION PERMETTANT D'INHIBER LA PROLIFERATION CELLULAIRE ET L'ANGIOGENESE

(30) Priority: 17.04.2006 US 406467; 18.04.2005 US 672689 P; 05.01.2006 US 756391 P; 31.01.2006 US 764039 P
(43) Date of publication of application: 16.01.2008
(73) Proprietor: SRI International, Menlo Park, CA 94025 (US)
(72) Inventor: LADEROUTE, Keith, R., Palo Alto, California 94301 (US); CALAOAGAN, Joy, M., San Jose, California 95148 (US); CHAO, Wan-Ru, Sunnyvale, California 94087 (US); PETERS, Richard, H., San Jose, California 95136 (US); HOBBS, Peter, D., Moss Beach, California 94038 (US); TANABE, Masato, Palo Alto, California 94303 (US); AMIN, Khalid, Mission, KS 66202 (US)
(74) Representative: Mallalieu, Catherine Louise
(86) International application number: PCT/US2006/014825
(87) International publication number: WO 2006/113842

(56) References cited:
- WO-A-99/33858
- WO-A2-99/33859
- US-A1- 2003 153 543
- US-A1- 2005 014 737
- US-B1- 6 503 896
- US-B2- 6 548 491
- MOOBERRY S.L.: 'New insights into 2-methoxyestradlol a promising antiangiogenic and antitumor angent' CURRENT OPINION IN ONCOLOGY vol. 15, 2003, pages 425 - 430, XP008073765

## Description

### TECHNICAL FIELD

This invention relates generally to inhibition of HIF-1 and angiogenesis, and more particularly relates to the use of certain substituted 1,3,5(10)-estratrienes as HIF-1 and angiogenesis inhibitors. The invention also pertains to pharmaceutical compositions for treating conditions, diseases and disorders that are responsive to administration of a HIF-1 or an angiogenesis inhibitor.

### BACKGROUND ART

Angiogenesis is the fundamental process by which new blood vessels are formed. The process involves the migration of vascular endothelial cells into tissue, followed by condensation of the cells into vessels. Angiogenesis may occur as the result of a natural condition, or may be induced by an angiogenic agent. The angiogenesis process is essential to a variety of normal physiological functions, such as *in utero* formation of tissues and organs, fetal development and wound repair. Although the process is not completely understood, it involves a complex interplay of compounds that stimulate and compounds that inhibit the growth and migration of endothelial cells, the primary cells of the capillary blood vessels. Under normal conditions, the angiogenesis process maintains the microvasculature in a quiescent state (i.e., without capillary growth) for prolonged periods that can last for several years or more.

Although angiogenesis is a highly regulated process under normal conditions, many disorders and diseases are driven by persistent unregulated angiogenesis. Unregulated angiogenesis can either cause a particular disorder or disease directly or exacerbate an existing pathological condition, such that neovascularization becomes a detrimental, and not a desirable, condition as it would be in wound healing. For example, ocular neovascularization has been implicated as the most common cause of blindness and underlies the pathology of numerous adverse conditions of the eye. With some previously existing conditions such as arthritis, newly formed capillary blood vessels invade the joints and destroy cartilage. In diabetes, new capillaries formed in the retina invade the vitreous humor, causing bleeding and blindness.

Both the growth and metastasis of solid tumors are also angiogenesis-dependent. It has been shown, for example, that tumors that enlarge to greater than 2 mm in diameter must obtain their own blood supply and do so by inducing the growth of new capillary blood vessels. After these new blood vessels become embedded in the tumor, they provide nutrients and growth factors essential for tumor growth as well as a means for tumor cells to enter the circulation and metastasize to distant sites, such as liver, lung or bone (Folkman (1986) Cancer Res. 46(2):467-473). Clearly, then, the prevention or reduction of angiogenesis is desirable for treating cancer and other angiogenesis-related disorders and diseases.

One particular arena in which inhibition of tumor angiogenesis is a critically important goal is in the treatment of prostate cancer (PC). PC is the most commonly diagnosed noncutaneous malignancy in the United States, and although cure is possible for men with localized PC using local treatment modalities, no curative treatment exists for more advanced disease. An effective treatment for late-stage PC must overcome the anti-apoptotic drug resistance mechanisms that are considered to operate in androgen-independent disease. Tumor growth at the primary site of PC is not usually lethal, but prevention of metastasis to distal sites and proliferation in metastatic foci is critical to survival. Primary therapy for advanced PC consists of androgen ablation, a strategy that controls metastatic PC in > 75% of men treated for a period of time. Unfortunately, however, all men with metastatic PC eventually develop androgen-independent (AI) disease. Hormone-refractory PC (HRPC) evolves from a subset of metastatic PC cells that acquire the ability to activate survival pathways and proliferate by androgen receptor (AR)-mediated signaling pathways without androgenic stimulation. Once men develop HRPC, treatment is directed at palliation of symptoms, and the median survival is in the range of 6 to 12 months. A recently approved combination therapy (docetaxel + prednisone) for HRPC increased survival, but only by 2-4 months.

Hypoxia-inducible factor 1 (HIF-1) is a heterodimeric protein that is made up of two proteins: HIF-1α and HIF-1β. HIF-1 regulates gene expression in cells exposed to low oxygen in both physiological and pathophysiological situations.

HIF-1 activates the transcription of many genes that code for proteins that are involved in angiogenesis, glucose metabolism, cell proliferation/survival and invasion/metastasis. HIF-1 also plays an essential role in surprisingly diverse normal tissue processes, including wound healing, bone development, adipogenesis, mammary gland development, and inflammation (Elson DA et al. Cancer Res 60: 6189-6195 (2000); Schipani E. et al. Genes Dev 15: 2865-2876 (2001); Yun Z et al Dev Cell 2: 331-341 (2002); Cramer T et al. Cell 112: 645-657 (2003); Seagroves TN et al Development 130: 1713-1724 (2003)).

HIF-1α protein synthesis is regulated by activation of the phosphatidylinositol 3-kinase (PI3K) and ERK mitogen activated protein kinase (MAPK) pathways. These pathways can be activated by signaling via receptor tyrosine kinases, non-receptor tyrosine kinases, or G-protein coupled receptors.

HIF-1α protein degradation is regulated by oxygen-dependent prolyl hydroxylation, which targets the protein for ubiquitylationby E3 ubiquitin-protein ligases. These ligases contain the Von Hippel lindau tumor suppressor protein (VHL), which binds specifically to hydroxylated HIF-1α. Ubiquitylated HIF1α is rapidly degraded by the proteasome.

HIF-1α is overexpressed in human cancers as a result of intratumoral hypoxia as well as genetic alterations, such as gain-of function mutations in oncogenes (such as ERBB2) and loss of function mutations in tumor suppressor genes (such as VHL and PTEN). HIF1α overexpression is associated with treatment failure and increased mortality (Semenza GL Nat Rev Cancer 3:721-32 (2003)).

The angiogenesis inhibitors that have been developed to date have been associated with significant disadvantages. For example, suramin is a potent angiogenesis inhibitor, but, at doses required to reach antitumor activity, causes severe systemic toxicity in humans. Other compounds, such as retinoids and interferons appear safe for human use but have only a weak anti-angiogenic effect. Still other compounds may be difficult or costly to make. In view of these problems, there exists an ongoing need for more effective methods and compositions for inhibiting angiogenesis. An ideal angiogenesis inhibitor would:
be sufficiently potent so that angiogenesis is inhibited at doses that do not result in significant systemic toxicity;
exhibit substantial pro-apoptotic activity;
be orally active, i.e., exhibit sufficient oral bioavailability that parenteral administration is unnecessary;
be capable of use in combination with another antiangiogenic or antiproliferative agents, and perhaps even demonstrate a synergistic - i.e., better than additive - effect when used with a second agent; and
be straightforward and cost-effective to synthesize.

The present invention provides use of compounds that exhibit some or all of the foregoing characteristics for the preparation of a medicament for inhibiting proliferation and angiogenesis. U.S. Patent Nos. 6,054,446, 6,281,205, 6,455,517, 6,503,896, 6,548,491, and 6,747,018 to Tanabe et al. (commonly assigned herewith to SRI International, Menlo Park, CA), describe a family of novel steroid compounds as anti-estrogenic agents and useful for treating estrogen-dependent disorders (i.e., conditions or diseases that are estrogen-induced or estrogen-stimulated), including cancers of the breast, ovaries, and uterus.

WO993385 discloses anti-estrogenic compounds which are useful to treat a variety of disorders, particularly estrogen-dependant disorders. Preferred compounds have 1,3,5-estratiene nucleus, and are substituted at the C-17 or C-11 position with a molecular moiety which renders the compounds effective to competitively block the binding of estrogen to its receptor. Particularly preferred compounds are 17-desoxy-1,3,5-estratienes. Therapeutic methods and pharmaceutical compositions are provided as well.

W09933858 discloses compounds useful as inhibitors of estrone sulfatase. The compounds have structural formula (I) wherein r1 is an optional double bond, R¹ and R² are selected from the group consisting of hydrogen and lower alkyl, or together form a cyclic substituent (II), wherein Q is NH, O or CH₂, and the other various substituents are as defined herein. Pharmaceutical compositions and methods for using the compounds of formula (I) to treat estrogen-disorders are provided as well.

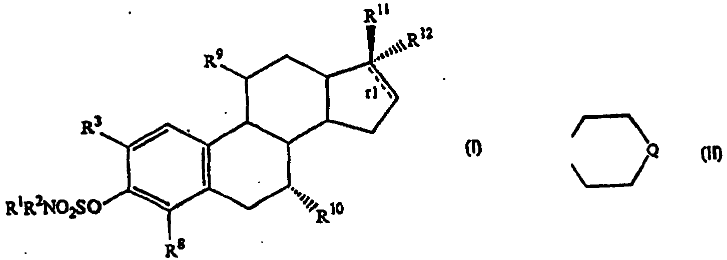

US2005/0014737 discloses compositions and methods for treating mammalian disease characterized by undesirable angiogenesis by administering compounds of the general formula:

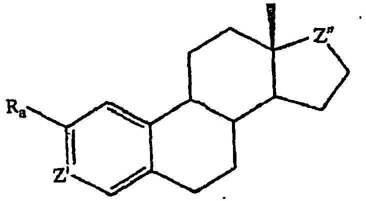

### DISCLOSURE OF THE INVENTION

It has now been discovered that certain compounds described in the aforementioned patents as anti-estrogenic agents are, quite unexpectedly, also potent inhibitors of HIF-1 and angiogenesis, and are particularly useful in the treatment of cancers that are not hormone-dependent, such as advanced prostate cancer.

The invention relates to use of a compound of formula (I) or (III) for the preparation of a medicament for treating a medical condition that involves angiogenesis. The medical condition is one that can be treatable by at least partial inhibition of angiogenesis. Generally, the condition is a disease or disorder associated with or resulting from unregulated angiogenesis. Such conditions include, without limitation: cancer, including lung cancer, brain cancer, and prostate cancer, particularly non-androgen dependent prostate cancer; conditions associated with detrimental neovascularization, such as ocular disorders associated with ocular neovascularization, including neovascular glaucoma, corneal graft neovascularization, retrolental fibroplasia, and diabetic retinopathy; and chronic inflammatory conditions such as psoriasis and rheumatoid arthritis.

The invention also relates to use of a compound of formula (I) or (III) for the preparation of a medicament for treating a medical condition that involves HIF-1 overexpression, such as angiogenic conditions, glucose metabolism anomalies, and cell proliferation/survival and invasion/metastasis conditions, among others.

In accordance with a first aspect of the invention there is provided a use of a compound of formula (I) or (III) 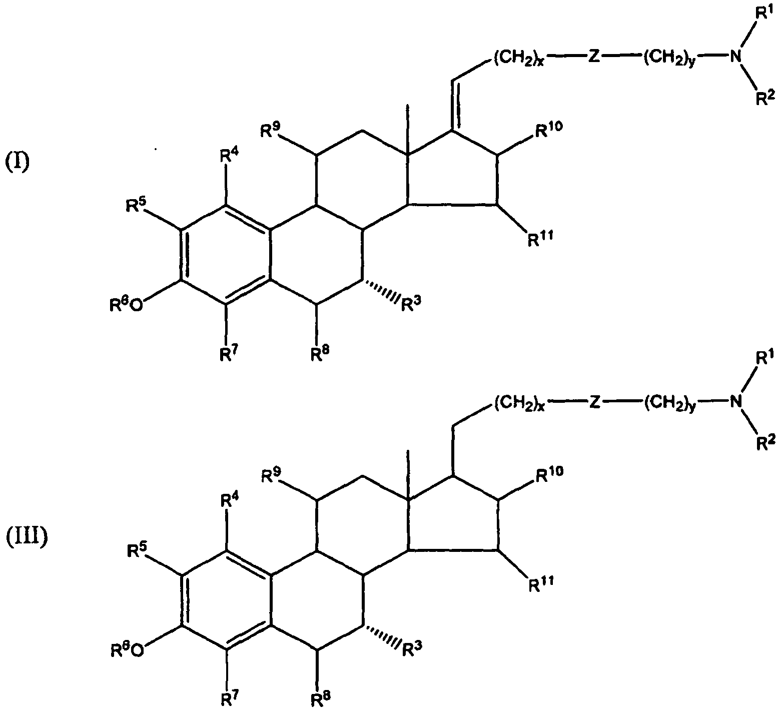 wherein:
Z is -O-(L)_{q}-_{;} -S-(L)q-, or -NR¹²-(L)_{q}- wherein q is zero or 1, L is monocyclic aryl optionally substituted with up to 4 substituents independently selected from hydrogen, hydroxyl, C₁-C₆ alkoxy, C₁-C₆ alkyl, halo, amino, and C₁-C₆ alkyl-substituted amino, wherein any two adjacent substituents on L may be taken together to form an optionally substituted cyclic structure, and R¹² is hydrogen or C₁-C₆ alkyl;
x is an integer in the range of 1 to about 6 inclusive;
when q is zero, y is an integer in the range of 2 to about 6 inclusive, and when q is 1, y is an integer in the range of 1 to about 6 inclusive;
R¹ and R² are independently selected from hydrogen and C₁-C₆ alkyl, or can be taken together to form an optionally substituted nitrogen heterocycle containing zero to two additional heteroatoms;
R³ is selected from hydrogen, hydroxyl, C₁-C₆ alkoxy, halo, C₁-C₃ alkyl, C₂-C₃ alkenyl, monocyclic aryl, and monocyclic aryl-substituted C₁-C₃ alkyl;
R⁴ is hydrogen or C₁-C₆ alkyl;
R⁵ is selected from hydrogen, C₁-C₆ alkoxy, halo, cyano, C₁-C₆ alkyl, and C₂-C₆ alkenyl;
R⁶ is selected from hydrogen, C₁-C₆ alkyl, C₂-C₁₂ acyl, and -SO₂NH₂;
R⁷ is selected from hydrogen, halo, C₁-C₆ alkyl, C₂-C₆ alkenyl, -OR¹³ and - SR¹³ where R¹³ is C₁-C₆ alkyl, C₂-C₆ acyl, or aryl;
R⁸ is hydrogen, C₁-C₆ alkoxy, or hydroxyl;
R⁹ is selected from hydrogen, hydroxyl, C₁-C₆ alkyl, C₂-C₆ alkenyl, aryl, and alkaryl; and
R¹⁰ and R¹¹ are independently selected from hydrogen, C₁-C₆ alkoxy, and C₁-C₆ alkyl,
or a pharmaceutically acceptable salt thereof for the preparation of a medicament for treating a disease or disorder involving angiogenesis or HIF-1 overexpression in a patient.

The use of compounds of formula (I) and formula (III) for the preparation of a medicament are useful in the invention as well. For example, in one embodiment there is disclosed the use of a compound of formula (I) or (III) for the preparation of a medicament for inhibiting HIF-1 overexpression or angiogenic activity in mammalian tissue, comprising contacting the tissue with a compound of formula (I) or (III). In another embodiment, there is disclosed the use of a compound of formula (I) or (III) for the preparation of a medicament for inhibiting the proliferation of mammalian endothelial cells, comprising contacting such cells with a compound of formula (I) or (III). In yet another embodiment, there is disclosed the use of a compound of formula (I) or (III) for the preparation of a medicament for inhibiting the proliferation of mammalian cells that overexpress HIF-1, the method comprising contacting such cells with a compound of formula (I) or (III).

In a further embodiment, a compound of formula (I) or (III) is used in the preparation of a medicament for treatment of prostate cancer, including non-androgen dependent prostate cancer. In a related embodiment, there is disclosed the use of a compound of formula (I) or (III) for the preparation of a medicament for inducing apoptosis in cancer cells, comprising contacting such cells with a compound having the structure of formula (I) or (III).

In a further embodiment, a compound of formula (I) or (III) is used in the preparation of a medicament to effect cell cycle arrest of cancer cells in the G1 phase, i.e., prior to the S phase in which DNA synthesis occurs.

A method is also described for lowering the effective dose of an anticancer agent, i.e., for reducing the effective dose below the minimum effective dose required when using the agent in a monotherapy regimen, wherein the method comprises administering the anticancer agent with a compound of formula (I) or (III).

It will be appreciated that compounds of formula (IV) are analogous to compounds of formula (I) but have a single bond linking the C 17 and C20 carbon atoms, while compounds of formula (II) have a double bond linking C17 and C20.

The invention additionally provides a composition of matter in the form of an orally administrable pharmaceutical composition, the composition comprising a carrier suitable for incorporation into an oral dosage form and a therapeutically effective amount of a compound having the structure of formula (I), wherein x, y, Z, L, q, and R¹ through R¹³ are selected such that the molecular weight of the compound is at most about 750.

### DETAILED DESCRIPTION OF THE PREFERRED EMBODIMENTS

### DEFINITION AND NOMENCLATURE:

Unless otherwise indicated, the invention is not limited to specific compounds, substituents, pharmaceutical compositions, modes of administration or the like, as such may vary. It is also to be understood that the terminology used herein is for the purpose of describing particular embodiments only and is not intended to be limiting.

As used in the specification and the appended claims, the singular forms "a," "an," and "the" include plural referents unless the context clearly dictates otherwise. Thus, for example, reference to "a substituent" includes a single substituent as well as two or more substituents that may be the same or different, reference to "a compound" encompasses a combination or mixture of different compounds as well as a single compound, reference to "a pharmaceutically acceptable carrier" includes two or more such carriers as well as a single carrier, and the like.

In this specification and in the claims that follow, reference will be made to a number of terms, which shall be defined to have the following meanings:

As used herein, the phrase "having the formula" or "having the structure" is not intended to be limiting and is used in the same way that the term "comprising" is commonly used.

The term "alkyl" as used herein refers to a branched or unbranched saturated hydrocarbon group typically although not necessarily containing 1 to about 24 carbon atoms, such as methyl, ethyl, *n*-propyl, isopropyl, *n*-butyl, isobutyl, *t*-butyl, octyl, decyl, and the like, as well as cycloalkyl groups such as cyclopentyl, cyclohexyl, and the like. Generally, although again not necessarily, alkyl groups herein contain 1 to about 18 carbon atoms, preferably 1 to about 12 carbon atoms. The term "lower alkyl" intends an alkyl group of 1 to 6 carbon atoms. Preferred lower alkyl substituents contain 1 to 3 carbon atoms, and particularly preferred such substituents contain 1 or 2 carbon atoms (i.e., methyl and ethyl). "Substituted alkyl" refers to alkyl substituted with one or more substituent groups, and the terms "heteroatom-containing alkyl" and "heteroalkyl" refer to alkyl in which at least one carbon atom is replaced with a heteroatom, as described in further detail *infra.* If not otherwise indicated, the terms "alkyl" and "lower alkyl" include linear, branched, cyclic, unsubstituted, substituted, and/or heteroatom-containing alkyl or lower alkyl, respectively.

The term "alkenyl" as used herein refers to a linear, branched or cyclic hydrocarbon group of 2 to about 24 carbon atoms containing at least one double bond, such as ethenyl, *n*-propenyl, isopropenyl, *n*-butenyl, isobutenyl, octenyl, decenyl, tetradecenyl, hexadecenyl, eicosenyl, tetracosenyl, and the like. Generally, although again not necessarily, alkenyl groups herein contain 2 to about 18 carbon atoms, preferably 2 to 12 carbon atoms. The term "lower alkenyl" intends an alkenyl group of 2 to 6 carbon atoms, and the specific term "cycloalkenyl" intends a cyclic alkenyl group, preferably having 5 to 8 carbon atoms. The term "substituted alkenyl" refers to alkenyl substituted with one or more substituent groups, and the terms "heteroatom-containing alkenyl" and "heteroalkenyl" refer to alkenyl in which at least one carbon atom is replaced with a heteroatom. If not otherwise indicated, the terms "alkenyl" and "lower alkenyl" include linear, branched, cyclic, unsubstituted, substituted, and/or heteroatom-containing alkenyl and lower alkenyl, respectively.

The term "alkoxy" as used herein intends an alkyl group bound through a single, terminal ether linkage; that is, an "alkoxy" group may be represented as -O-alkyl where alkyl is as defined above. A "lower alkoxy" group intends an alkoxy group containing 1 to 6 carbon atoms, and includes, for example, methoxy, ethoxy, n-propoxy, isopropoxy, t-butyloxy, etc. Preferred lower alkoxy substituents contain 1 to 3 carbon atoms, and particularly preferred such substituents contain 1 or 2 carbon atoms (i.e., methoxy and ethoxy). An alkoxy substituent may be taken together with a second substituent, e.g., an adjacent substituent on a cyclic structure, to form a cyclic ether, e.g., a [1,4] dioxane, a [1,3] dioxolane, or a tetrahydropyranyl ring. The terms "alkenyloxy" and "alkynyloxy" are defined in an analogous manner.

The term "aryl" as used herein, and unless otherwise specified, refers to an aromatic substituent containing a single aromatic ring or multiple aromatic rings that are fused together, directly linked, or indirectly linked (such that the different aromatic rings are bound to a common group such as a methylene or ethylene moiety). Preferred aryl groups contain 5 to 24 carbon atoms, and particularly preferred aryl groups contain 5 to 14 carbon atoms. Exemplary aryl groups contain one aromatic ring or two fused or linked aromatic rings, e.g., phenyl, naphthyl, biphenyl, diphenylether, diphenylamine, benzophenone, and the like. "Substituted aryl" refers to an aryl moiety substituted with one or more substituent groups, and the terms "heteroatom-containing aryl" and "heteroaryl" refer to aryl substituent, in which at least one carbon atom is replaced with a heteroatom, as will be described in further detail *infra.* If not otherwise indicated, the term "aryl" includes unsubstituted, substituted, and/or heteroatom-containing aromatic substituents.

The term "alkaryl" refers to an aryl group with an alkyl substituent, and the term "aralkyl" refers to an alkyl group with an aryl substituent, wherein "aryl" and "alkyl" are as defined above. Preferred aralkyl groups contain 6 to 24 carbon atoms, and particularly preferred aralkyl groups contain 6 to 16 carbon atoms. Examples of aralkyl groups include, without limitation, benzyl, 2-phenyl-ethyl, 3-phenyl-propyl, 4-phenyl-butyl, 5-phenyl-pentyl, 4-phenylcyclohexyl, 4-benzylcyclohexyl, 4-phenylcyclohexylmethyl, 4-benzylcyclohexylmethyl, and the like. Alkaryl groups include, for example, p-methylphenyl, 2,4-dimethylphenyl, p-cyclohexylphenyl, 2,7-dimethylnaphthyl, 7-cyclooctylnaphthyl, 3-ethyl-cyclopenta-1,4-diene, and the like. The terms "alkaryloxy" and "aralkyloxy" refer to substituents of the formula -OR wherein R is alkaryl or aralkyl, respectively, as just defined.

The term "acyl" refers to substituents having the formula -(CO)-alkyl, -(CO)-aryl, or -(CO)-aralkyl, and the term "acyloxy" refers to substituents having the formula -O(CO)-alkyl, -O(CO)-aryl, or -O(CO)-aralkyl, wherein "alkyl," "aryl, and "aralkyl" are as defined above.

The term "cyclic" refers to alicyclic or aromatic substituents that may or may not be substituted and/or heteroatom containing, and that may be monocyclic, bicyclic, or polycyclic.

The term "alicyclic" is used in the conventional sense to refer to an aliphatic cyclic moiety, as opposed to an aromatic cyclic moiety, and may be monocyclic, bicyclic or polycyclic.

The terms "halo" and "halogen" are used in the conventional sense to refer to a chloro, bromo, fluoro or iodo substituent.

The term "heteroatom-containing" as in a "heteroatom-containing alkyl group" (also termed a "heteroalkyl" group) or a "heteroatom-containing aryl group" (also termed a "heteroaryl" group) refers to a molecule, linkage or substituent in which one or more carbon atoms are replaced with an atom other than carbon, e.g., nitrogen, oxygen, sulfur, phosphorus - or silicon, typically nitrogen, oxygen or sulfur, preferably nitrogen or oxygen. Similarly, the term "heteroalkyl" refers to an alkyl substituent that is heteroatom-containing, the term "heterocyclic" refers to a cyclic substituent that is heteroatom-containing, the terms "heteroaryl" and heteroaromatic" respectively refer to "aryl" and "aromatic" substituents that are heteroatom-containing, and the like. Examples of heteroalkyl groups include alkoxyaryl, alkylsulfanyl-substituted alkyl, N-alkylated amino alkyl, and the like. Examples of heteroaryl substituents include pyrrolyl, pyrrolidinyl, pyridinyl, quinolinyl, indolyl, pyrimidinyl, imidazolyl, 1,2,4-triazolyl, tetrazolyl, etc., and examples of heteroatom-containing alicyclic groups are pyrrolidino, morpholino, piperazino, piperidino, etc.

"Hydrocarbyl" refers to univalent hydrocarbyl radicals containing 1 to about 30 carbon atoms, preferably 1 to about 24 carbon atoms, more preferably 1 to about 18 carbon atoms, most preferably about 1 to 12 carbon atoms, including linear, branched, cyclic, saturated, and unsaturated species, such as alkyl groups, alkenyl groups, aryl groups, and the like. "Substituted hydrocarbyl" refers to hydrocarbyl substituted with one or more substituent groups, and the term "heteroatom-containing hydrocarbyl" refers to hydrocarbyl in which at least one carbon atom is replaced with a heteroatom. Unless otherwise indicated, the term "hydrocarbyl" is to be interpreted as including substituted and/or heteroatom-containing hydrocarbyl moieties.

When a functional group is termed "protected", this means that the group is in modified form to preclude undesired side reactions at the protected site. Suitable protecting groups for the compounds of the present invention will be recognized from the present application taking into account the level of skill in the art, and with reference to standard textbooks, such as Greene et al., Protective Groups in Organic Synthesis (New York: Wiley, 1991).

By "substituted" as in "substituted alkyl," "substituted aryl," and the like, as alluded to in some of the aforementioned definitions, is meant that in the alkyl, aryl, or other moiety, at least one hydrogen atom bound to a carbon (or other) atom is replaced with one or more non-hydrogen substituents. Examples of such substituents include, without limitation: functional groups such as halo, hydroxyl, sulfhydryl, C₁-C₂₄ alkoxy, C₂-C₂₄ alkenyloxy, C₅-C₂₄ aryloxy, acyl (including C₂-C₂₄ alkylcarbonyl (-CO-alkyl) and C₆-C₂₄ arylcarbonyl (-CO-aryl)), acyloxy (-O-acyl), C₂-C₂₄ alkoxycarbonyl (-(CO)-O-alkyl), C₆-C₂₄ aryloxycarbonyl

(-(CO)-O-aryl), halocarbonyl (-CO)-X where X is halo), C₂-C₂₄ alkylcarbonato (-O-(CO)-O-alkyl), C₆-C₂₄ arylcarbonato (-O-(CO)-O-aryl), carboxy (-COOH), carboxylato (-COO⁻), carbamoyl (-(CO)-NH₂), mono-(C₁-C₂₄ alkyl)-substituted carbamoyl (-(CO)-NH(C₁-C₂₄ alkyl)), di-(C₁-C₂₄ alkyl)-substituted carbamoyl (-(CO)-N(C₁-C₂₄ alkyl)₂), mono-(C₆-C₂₄ aryl)-substituted carbamoyl (-(CO)-NH-aryl), di-(C₆-C₂₄ aryl)-substituted carbamoyl (-(CO)-N(aryl)₂), di-N-(C₁-C₂₄ alkyl), N-(C₆-C₂₄ aryl)-substituted carbamoyl, thiocarbamoyl (-(CS)-NH₂), carbamido (-NH-(CO)-NH₂), cyano(-C≡N), isocyano (-N⁺≡C⁻), cyanato (-O-C≡N), isocyanato (-O-N⁺≡C⁻), isothiocyanato (-S-C≡N), azido (-N=N⁺=N⁻), formyl (-(CO)-H), thioformyl (-(CS)-H), amino (-NH₂), mono-(C₁-C₂₄alkyl). substituted amino, di-(C₁-C₂₄ alkyl)-substituted amino, mono-(C₅-C₂₄ aryl)-substituted amino, di-(C₅-C₂₄ aryl)-substituted amino, C₂-C₂₄ alkylamido (-NH-(CO)-alkyl), C₆-C₂₄ arylamido (-NH-(CO)-aryl), imino

(-CR=NH where R = hydrogen, C₁-C₂₄ alkyl, C₅-C₂₄ aryl, C₆-C₂₄ alkaryl, C₆-C₂₄ aralkyl, etc.), alkylimino (-CR=N(alkyl), where R = hydrogen, C₁-C₂₄ alkyl, C₅-C₂₄ aryl, C₆-C₂₄ alkaryl, C₆-C₂₄ aralkyl, etc.), arylimino (-CR=N(aryl), where R = hydrogen, C₁-C₂₄ alkyl, C₅-C₂₄ aryl, C₆-C₂₄ alkaryl, C₆-C₂₄ aralkyl, etc.), nitro (-NO₂), nitroso (-NO), sulfo (-SO₂-OH), sulfonato

(-SO₂-O⁻), C₁-C₂₄ alkylsulfanyl (-S-alkyl; also termed "alkylthio"), arylsulfanyl (-S-aryl; also termed "arylthio"), C₁-C₂₄ alkylsulfinyl (-(SO)-alkyl), C₅-C₂₄ arylsulfinyl (-(SO)-aryl), C₁-C₂₄ alkylsulfonyl (-SO₂-alkyl), C₅-C₂₄ arylsulfonyl (-SO₂-aryl), phosphono (-P(O)(OH)₂), phosphonato (-P(O)(O⁻)₂), phosphinato (-P(O)(O⁻)), phospho (-PO₂), and phosphino (-PH₂); and the hydrocarbyl moieties C₁-C₂₄ alkyl (preferably C₁-C₁₈ alkyl, more preferably C₁-C₁₂ alkyl, most preferably C₁-C₆ alkyl), C₂-C₂₄ alkenyl (preferably C₂-C₁₈ alkenyl, more preferably C₂-C₁₂ alkenyl, most preferably C₂-C₆ alkenyl), C₅-C₂₄ aryl (preferably C₅-C₁₄ aryl), C₆-C₂₄ alkaryl (preferably C₆-C₁₈ alkaryl), and C₆-C₂₄ aralkyl (preferably C₆-C₁₈ aralkyl).

In addition, the aforementioned functional groups may, if a particular group permits, be further substituted with one or more additional functional groups or with one or more hydrocarbyl moieties such as those specifically enumerated above. Analogously, the above-mentioned hydrocarbyl moieties may be further substituted with one or more functional groups or additional hydrocarbyl moieties such as those specifically enumerated.

When the term "substituted" appears prior to a list of possible substituted groups, it is intended that the term apply to every member of that group. For example, the phrase "substituted alkyl, alkenyl, and aryl" is to be interpreted as "substituted alkyl, substituted alkenyl, and substituted aryl." Analogously, when the term "heteroatom-containing" appears prior to a list of possible heteroatom-containing groups, it is intended that the term apply to every member of that group. For example, the phrase "heteroatom-containing alkyl, alkenyl, and aryl" is to be interpreted as "heteroatom-containing alkyl, substituted alkenyl, and substituted aryl."

"Optional" or "optionally" means that the subsequently described circumstance may or may not occur, so that the description includes instances where the circumstance occurs and instances where it does not For example, the phrase "optionally substituted" means that a non-hydrogen substituent may or may not be present on a given atom, and, thus, the description includes structures wherein a non-hydrogen substituent is present and structures wherein a non-hydrogen substituent is not present.

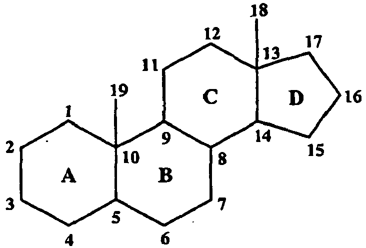

In describing the location of groups and substituents, the above numbering systems will be employed, to conform the numbering of the cyclopentanophenanthrene nucleus to the convention used by the IUPAC or Chemical Abstracts Service. The term "steroid" as used herein is intended to mean compounds having the aforementioned cyclopentanophenanthrene nucleus. The steroids of the invention are substituted 1,3,5(10) estratrienes, having as the core structure

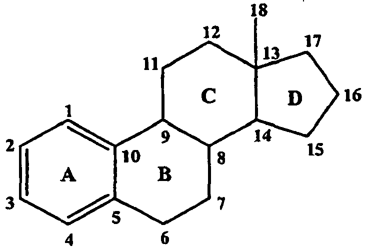

In the molecular structures herein, the used of bold and dashed lines to denote particular conformation of groups follows the IUPAC convention. A bond indicated by a broken line indicates that the group in question is below the general plane of the molecule as drawn (the "a" configuration), and a bond indicated by a bold line indicates that the group at the position in question is above the general plane of the molecule as drawn (the "β" configuration).

It should also be emphasized that certain compounds or molecular segments herein may contain one or more chiral centers and thus may be a racemic mixture (50-50) of isomers, a mixture of isomers where one isomer is present in excess, or a substantially pure isomer, "substantially pure" meaning that one isomer represents greater than 90%, preferably greater than 95%, more preferably greater than 99%, of a mixture of isomers. It is intended that for such chiral molecules the disclosure herein encompasses a mixture of isomers as well as a substantially pure isomer.

When an olefinic compound or molecular segment is drawn in the "E" or "Z" configuration, it is to be understood that the molecular structure is intended to encompass both alternatives, and that, therefore, an olefinic moiety drawn as substituted in the "E" configuration should also be interpreted as encompassing the "Z" configuration. For example, the C17-C20 double bond in compound (I) is indicated herein as substituted in the "E" configuration. The structure drawn is also intended to encompass the "Z" configuration, although the "E" configuration, in the compound as drawn, is generally preferred.

When referring to a compound of the invention as an active agent, applicants intend the term "compound" or "active agent" to encompass not only the specified molecular entity but also its pharmaceutically acceptable, pharmacologically active analogs, including, but not limited to, salts, esters, amides, prodrugs, conjugates, active metabolites, and other such derivatives, analogs, and related compounds.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. Thus, "treading" a patient with a compound of the invention includes prevention of a particular disorder or adverse physiological event in a susceptible individual as well as treatment of a clinically symptomatic individual by inhibiting or causing regression of a disorder or disease.

-By the terms "effective amount" and "therapeutically effective amount" of a compound of the invention is meant a sufficient amount of the drug or agent to provide the desired effect.

The term "dosage form" denotes any form of a pharmaceutical composition that contains an amount of active agent sufficient to achieve a therapeutic effect with a single administration. When the formulation is a tablet or capsule, the dosage form is usually one such tablet or capsule. The frequency of administration that will provide the most effective results in an efficient manner without overdosing will vary with the characteristics of the particular active agent, including both its pharmacological characteristics and its physical characteristics, such as hydrophilicity.

By "pharmaceutically acceptable" is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" derivative or analog, refers to a derivative or analog having the same type of pharmacological activity as the parent compound and approximately equivalent in degree.

### ANTIANGIOGENIC AND ANTI-HIF-1 COMPOUNDS:

In one embodiment, the steroidal antiangiogenic and anti HIF-1 compounds used in the present methods have the structure of formula (I) 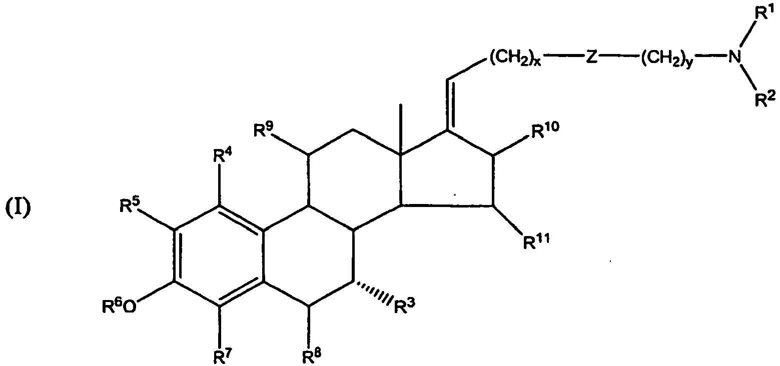 wherein the variables and substituents are as follows:

Z is -O-(L)_{q}-, -S-(L)q-, or -NR¹²-(L)_{q}- wherein q is zero or 1, L is monocyclic aryl optionally substituted with up to 4 substituents independently selected from hydrogen, hydroxyl, C₁-C₆ alkoxy (e.g., methoxy, ethoxy, and the like), C₁-C₆ alkyl (e.g., methyl, ethyl, n-propyl, isopropyl, t-butyl, n-hexyl, and the like), halo, amino, and C₁-C₆ alkyl-substituted amino (e.g., methylamino, ethylamino, isopropylamino, cyclohexylamino, and the like), wherein any two adjacent substituents on L may be taken together to form an optionally substituted cyclic structure, and R¹² is hydrogen or C₁-C₆ alkyl. The cyclic structure formed when adjacent substituents on L are linked may be, for example, a cyclic ether, e.g., a [1,4] dioxane, a [1,3] dioxolane, or a tetrahydropyranyl ring. When q is 1, such that L is present, preferred compounds are those wherein any substituents on L are C₁-C₆ alkoxy, e.g., methoxy, ethoxy, etc. In a particularly preferred embodiment, L is phenyl, 2-methoxyphenyl, 3-methoxyphenyl, 2,5-dimethoxyphenyl, or 2,6-dimethoxyphenyl. Z is preferably O, regardless of whether q is zero or 1.

The subscripts x and y may be the same or different, and are integers in the range of 1 to about 6 inclusive; however, when q is zero, y is an integer in the range of 2 to about 6 inclusive, and when q is 1, y may be an integer in the range of 1 to about 6 inclusive. In a preferred embodiment, x is 1 or 2 and y is 2. For those compounds of formula (I) in which q is 1, such that L is present, x and y are preferably 1. For those compounds of formula (II) in which q is zero, such that L is absent, x is preferably 1 and y is 2.

R¹ and R² are independently selected from hydrogen and C₁-C₆ alkyl, or can be taken together to form an optionally substituted nitrogen heterocycle containing zero to two additional heteroatoms. For instance, R¹ can be hydrogen, methyl, or ethyl, and R² can be methyl or ethyl, or R¹ and R² may be taken together to form a nitrogen heterocycle such as a pyrrolidino, morpholino, piperazino, or piperidino ring, optionally substituted with one or more substituents.

R³ is selected from hydrogen, hydroxyl, halo, C₁-C₃ alkyl, C₁-C₆ alkoxy, C₂-C₃ alkenyl, monocyclic aryl, and monocyclic aryl-substituted C₁-C₃ alkyl, and is preferably hydrogen or C₁-C₃ alkyl, most preferably hydrogen or methyl.

R⁴ is hydrogen or C₁-C₆ alkyl, preferably hydrogen.

R⁵ is selected from hydrogen, C₁-C₆ alkoxy, halo, cyano, C₁-C₆ alkyl, and C₂-C₆ alkenyl. Preferably R⁵ is hydrogen or C₁-C₆ alkoxy, optimally hydrogen or methoxy.

R⁶ is selected from hydrogen, C₁-C₆ alkyl, C₂-C₁₂ acyl, and -SO₂NH₂. The latter two substituents transform the compound into a "prodrug." Other prodrugs of the compound of formula (I) are also possible, and are encompassed by the methods and compositions of the invention.

R⁷ is selected from hydrogen, halo, C₁-C₆ alkyl, C₂-C₆ alkenyl, -OR¹³ and - SR¹³ where R¹³ is C₁-C₆ alkyl, C₂-C₆ acyl, C₂-C₆ acyloxy, or aryl. Typically, R⁷ is hydrogen, C₁-C₆ alkyl, or C₂-C₆ acyloxy.

R⁸ is hydrogen, C₁-C₆ alkoxy, or hydroxyl, preferably hydrogen.

R⁹ is selected from hydrogen, hydroxyl, C₁-C₆ alkyl, C₂-C₆ alkenyl, aryl, and alkaryl, and is preferably hydrogen.

R¹⁰ and R¹¹ are independently selected from hydrogen, C₁-C₆ alkoxy, and C₁-C₆ alkyl, and are preferably hydrogen.

In one important embodiment of the invention, compounds of formula (I) are provided that are sufficiently orally bioavailable to be administrable orally; in such compounds, x, y, Z, L, q, and R¹ through R¹³ are selected such that the molecular weight of the compound is at most about 750.

In one preferred subset of formula (I) compounds, R⁴, R⁵, R⁶, R⁷, R⁸, R⁹, R¹⁰, and R¹¹ are hydrogen, such that the compound has the structure of formula (II)

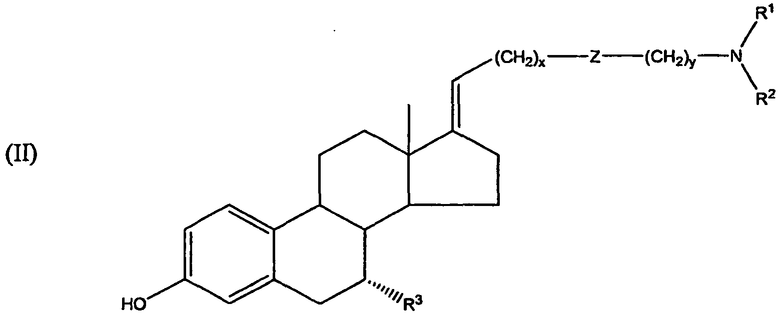

In one group of preferred such compounds, q is zero, such that Z is -O-, -S-, or -NR¹²-, and x is 1 or 2 and y is 2. Preferably, Z is O. R¹ and R² are as defined above, but preferably one of R¹ and R² is hydrogen, methyl, or ethyl, the other is methyl or ethyl, or R¹ and R² are taken together to form a nitrogen heterocycle such as a pyrrolidino, morpholino, piperazino, or piperidino ring, optionally substituted with one or more substituents. In these preferred compounds, R³ is hydrogen or methyl.

In formula (II), when x is 1, y is 2, R¹ and R² are methyl, R³ is methyl, q is zero, and Z is -O-, one exemplary compound of the invention is provided, having the structure of formula (**2**)

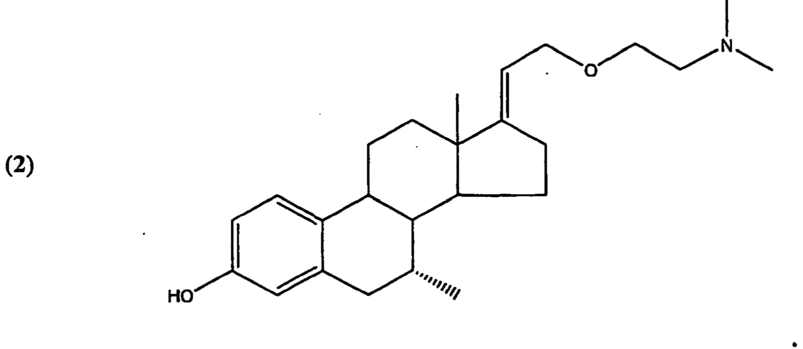

When x is 1, y is 1, R¹ and R² are ethyl, R³ is methyl, q is 1, Z is -O-L-, and L is 2-methoxyphenyl or phenyl, the following two exemplary compounds are provided:

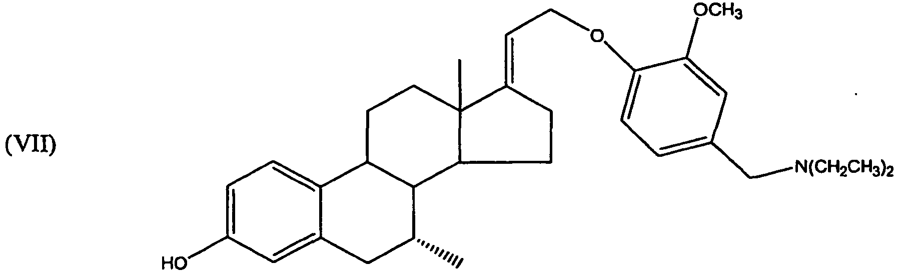

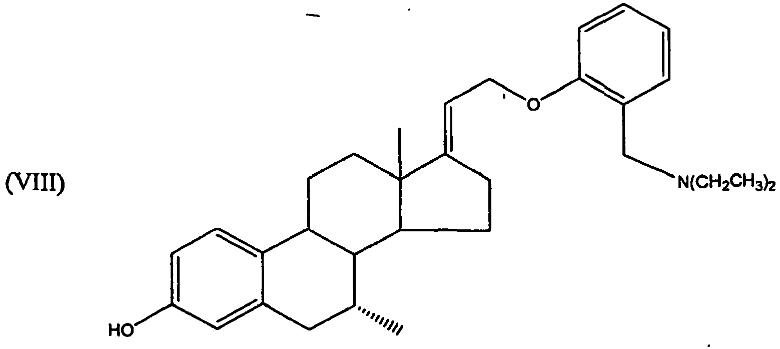

Other steroidal antiangiogenic and anti HIF-1 compounds useful in the present methods have the structure of formula (III) 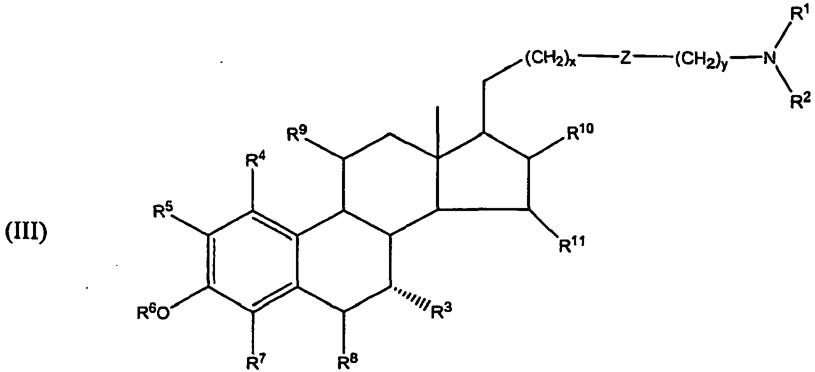 wherein x, y, Z, L, q, and R¹ through R¹³ are defined as for the variables and substituents of formula (I). It will be appreciated that compounds of formula (III) are analogous to compounds of formula (I) but have a single bond linking the C17 and C20 carbon atoms, while compounds of formula (II) have a double bond linking C17 and C20.

In one preferred subset of formula (III) compounds, R⁴, R⁵, R⁶, R⁷, R⁸, R⁹, R¹⁰, and R¹¹ are hydrogen, such that the compound has the structure of formula (IV)

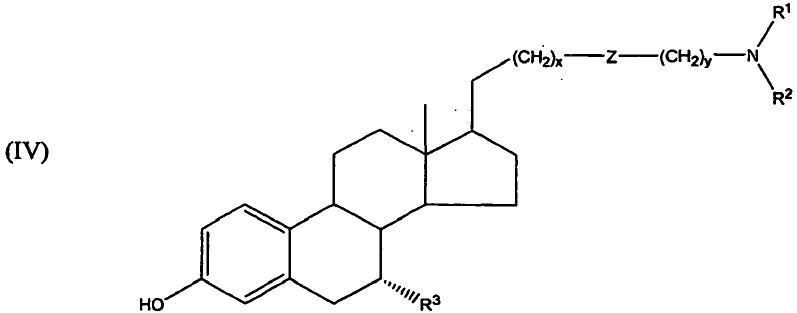

In particularly preferred such compounds, q is 1, Z is -O-L-, L is 2-methoxyphenyl, x is 1, and y is 1. R¹ and R² are as defined above, but are preferably methyl or ethyl, or are taken together to form a nitrogen heterocycle such as a pyrrolidino, morpholino, piperazino, or piperidino ring, optionally substituted with one or more substituents. R³ is hydrogen or methyl. One representative such compound, having the structure of formula (V), is wherein R¹ and R² are ethyl and R³ is methyl:

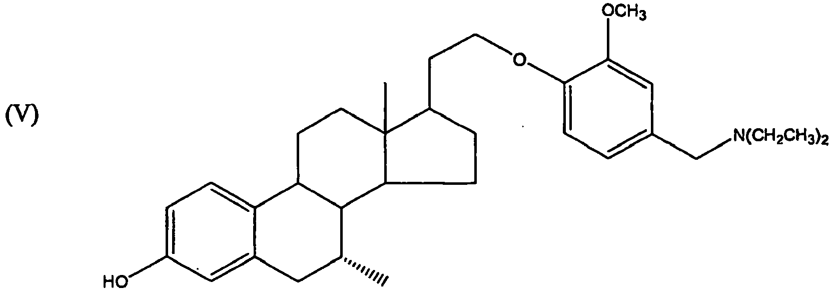

These compounds may be synthesized in high yield using relatively simple, straightforward methods. Synthesis of one representative compound encompassed by structural formula (I) is described in detail in Example 1, and preparation of the citrate salt of the compound is described in Example 2. Syntheses of many other formula (I) compounds are set forth in U.S. Patent No. 6,281,205 to Tanabe et al. (of common assignment herewith to SRI International, Menlo Park, California), which discloses the use of the compounds as anti-estrogenic agents, and which is herein incorporated by reference. The aforementioned patent also provides the syntheses of numerous compounds encompassed by structural formula (III). Those of ordinary skill in the art can synthesize other compounds of formulae (I) and (III) by modifying the synthesis set forth in Example 1 herein or the syntheses set forth in U.S. Patent No. 6,281,205 to Tanabe et al., according to methods known in the art or described in the pertinent texts and literature. For further information concerning the synthesis of compounds having a sulfamate group at the 3-position, i.e., at R⁶, reference may be had to U.S. Patent No. 6,046,186 to Tanabe et al., entitled "Estrone Sulfamate Inhibitors of Estrone Sulfatase, and Associated Pharmaceutical Compositions and Methods of Use" (also of common assignment herewith), which is herein incorporated by reference.

Any of the compounds just described may be in the form of a salt, ester, amide, prodrug, active metabolite, analog, or the like, provided that the salt, ester, amide, prodrug, active metabolite or analog is pharmaceutically acceptable and pharmacologically active in the present context. Salts, esters, amides, prodrugs, active metabolites, analogs, and other derivatives of the active agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992).
For example, acid addition salts may be prepared from a free base (the terminal amino group on the C17 substituent, in the present compounds) using conventional methodology involving reaction of the free base with an acid. Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt may be reconverted to the free base by treatment with a suitable base. Conversely, preparation of basic salts of any acidic moieties that may be present may be carried out in a similar manner using a pharmaceutically acceptable base such as sodium hydroxide, potassium hydroxide, ammonium hydroxide, calcium hydroxide, trimethylamine, or the like. Preparation of esters involves reaction of a hydroxyl group with an esterification reagent such as an acid chloride. Amides may be prepared from esters, using suitable amine reactants, or they may be prepared from an anhydride or an acid chloride by reaction with ammonia or a lower alkyl amine. Prodrugs, conjugates, and active metabolites may also be prepared using techniques known to those skilled in the art or described in the pertinent literature. Prodrugs and conjugates are typically prepared by covalent attachment of a moiety that results in a compound that is therapeutically inactive until modified by an individual's metabolic system.
Preferred analogs herein are acid addition salts formed by association of the tertiary amino group -NR¹R² and an acid as set forth above.

### USE AND ADMINISTRATION:

The compounds described herein are useful in the inhibition of angiogenesis and HIF-1, particularly persistent and uncontrolled HIF-1 overexpression and angiogenesis as associated with many adverse physiological conditions. In a first embodiment, use of a compound of formula (I) or (III) for the preparation of a medicament for treating a medical condition that is associated with angiogenesis or HIF-1 overexpression is provided. The treatment involves administering a therapeutically effective amount of a compound of formula (I) (in a preferred embodiment) or a compound of formula (III) (in another embodiment) to the patient. By "patient" is meant a mammalian individual, generally human.

The compounds can be administered to a patient by themselves or in pharmaceutical compositions in which they are mixed with a suitable carrier or excipient. Compounds of the invention may also be administered in combination, in which case they may be administered separately, in different dosage forms, or simultaneously, either in one dosage form or in two different dosage forms. Compounds may also be administered in combination with other antiangiogenic agents, antiproliferative agents, or other types of agents as may be deemed appropriate for a particular purpose by a prescribing physician. Combination therapy of particular interest involves administering a compound of the invention in conjunction with conventional chemotherapy, i.e., in a dosage regimen that includes administration of an anticancer agent such as a taxane, e.g., paclitaxel, docetaxel, analogs thereof, or the like. It has been found that administering a compound as described herein with another anticancer agent can reduce the required dosage of the agent and thus reduce the many side effects of such drugs.

Pharmaceutical formulations suitable for use in conjunction with the present invention include compositions wherein the active agent is contained in a "therapeutically effective" amount, i.e., in an amount effective to achieve its intended purpose, such as anti-angiogenesis, and anti-HIF-1. Determination of a therapeutically effective amount for any particular antiangiogenic or anti HIF-1 agent of the invention is well within the capability of those skilled in the art. That is, for any of the present compounds, a therapeutically effective dose can be estimated initially from cell culture assays. For example, a dose can be formulated to achieve a circulating concentration range that includes an IC₅₀ value as determined in cell culture. Such information can be used to more accurately determine useful doses in humans.

Toxicity and therapeutic efficacy of the compounds described herein can be determined by standard pharmaceutical procedures in cell cultures or experimental animals, e.g., procedures used for determining the maximum tolerated dose (MTD), the EDso, which is the effective dose to achieve 50% of maximal response, and the therapeutic index (TI), which is the ratio of the MTD to the ED₅₀. Obviously, compounds with high TIs are the most preferred compounds herein, and preferred dosage regimens are those that maintain plasma levels of the active agent at or above a minimum concentration to maintain the desired therapeutic effect. Dosage will, of course, also depend on a number of factors, including the particular compound, the site of intended delivery, the route of administration, and other pertinent factors known to the prescribing physician. Generally, however, dosage will be in the range of approximately 0.1 µg/kg/day to 100 mg/kg/day, more typically in the range of about 1.0 mg/kg/day to 10 mg/kg/day.
The compounds of the invention are useful as antiangiogenic and anti HIF-1 agents, and find utility in inhibiting angiogenesis and proliferation *per se,* e.g., by introduction of a compound of the invention into mammalian tissue (skin tissue, eye tissue, a tumor, etc.) to inhibit HIF-1, angiogenesis or proliferation therein, and by inhibiting the proliferation of endothelial cells associated with a tissue of interest (which may be cells comprising a tissue of interest, exogenous cells introduced into a tissue, or neighboring cells not within the tissue) by contacting the cells with a compound of the invention. Further, because the antiangiogenic and anti HIF-1 agents herein have been demonstrated to achieve cell cycle arrest in the G1 phase, i.e., prior to the S phase in which DNA synthesis occurs, the invention also provides a method for using the presently disclosed compounds to effect cell cycle arrest of cancer cells in the G1 phase. The agents described herein are also useful in inducing apoptosis, and are therefore useful in treating disorders responsive to the induction of apoptosis, particularly cancer. Other such disorders are well known and may be readily ascertained by one of ordinary skill in the art and/or by reference to the pertinent literature.
In addition to their utility in a method for inhibiting angiogenesis and overexpressed HIF-1 *per se,* the present agents are also useful for treating conditions, diseases, and disorders in which HIF-1, proliferation, and angiogenesis have been found to have a role, as indicated above. These conditions, diseases, and disorders include, without limitation: cancer, including lung cancer, brain cancer, and prostate cancer, particularly non-androgen dependent prostate cancer; conditions associated with detrimental neovascularization, such as ocular disorders associated with ocular neovascularization, including neovascular glaucoma, corneal graft neovascularization, retrolental fibroplasia, and diabetic retinopathy; and chronic inflammatory conditions such as psoriasis and rheumatoid arthritis.
The compound is generally administered in a pharmaceutically acceptable formulation as described *infra*.

Administration of the HIF-1 and angiogenesis inhibitor of the invention may be carried out using any appropriate mode of administration. Thus, administration can be, for example, oral, parenteral, transdermal, transmucosal (including rectal and vaginal), sublingual, by inhalation, or via an implanted reservoir in a dosage form. The term "parenteral" as used herein is intended to include, for example, subcutaneous, intravenous, and intramuscular injection.

Depending on the intended mode of administration, the pharmaceutical formulation may be a solid, semi-solid or liquid, such as, for example, a tablet, a capsule, a caplet, a liquid, a suspension, an emulsion, a suppository, granules, pellets, beads, a powder, or the like, preferably in unit dosage form suitable for single administration of a precise dosage. Suitable pharmaceutical compositions and dosage forms may be prepared using conventional methods known to those in the field of pharmaceutical formulation and described in the pertinent texts and literature, e.g., in Remington: The Science and Practice of Pharmacy (Easton, PA: Mack Publishing Co., 1995). For those compounds that are orally active, i.e., demonstrate sufficient oral bioavailability such that a safe but therapeutically effective dose can be administered orally, oral drug administration is preferred. In general, of the angiogenesis and proliferation inhibitors described herein, those compounds having a molecular weight of at most about 750 are orally active and thus orally administrable. Oral dosage forms for administration of these compounds include tablets, capsules, caplets, solutions, suspensions and syrups, and may also comprise a plurality of granules, beads, powders or pellets that may or may not be encapsulated. Preferred oral dosage forms are tablets and capsules.

Tablets may be manufactured using standard tablet processing procedures and equipment. In addition to the active agent, tablets will generally contain inactive, pharmaceutically acceptable carrier materials such as binders, lubricants, disintegrants, fillers, stabilizers, surfactants, coloring agents, and the like. Binders are used to impart cohesive qualities to a tablet, and thus ensure that the tablet remains intact. Suitable binder materials include, but are not limited to, starch (including corn starch and pregelatinized starch), gelatin, sugars (including sucrose, glucose, dextrose, and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, microcrystalline cellulose, ethyl cellulose, hydroxyethyl cellulose, and the like), and Veegum. Lubricants are used to facilitate tablet manufacture, promoting powder flow and preventing particle capping (i.e., particle breakage) when pressure is relieved. Disintegrants are used to facilitate disintegration of the tablet, and are generally starches, clays, celluloses, algins, gums, or crosslinked polymers. Fillers include, for example, materials such as silicon dioxide, titanium dioxide, alumina, talc, kaolin, powdered cellulose, and microcrystalline cellulose, as well as soluble materials such as mannitol, urea, sucrose, lactose, dextrose, sodium chloride, and sorbitol. Stabilizers, as well known in the art, are used to inhibit or retard drug decomposition reactions that include, by way of example, oxidative reactions.

Capsules are also preferred oral dosage forms for those angiogenesis and HIF-1 inhibitors that are orally active, in which case the active agent-containing composition may be encapsulated in the form of a liquid or solid (including particulates such as granules, beads, powders or pellets). Suitable capsules may be either hard or soft, and are generally made of gelatin, starch, or a cellulosic material, with gelatin capsules preferred. Two-piece hard gelatin capsules are preferably sealed, such as with gelatin bands or the like. See, for example, *Remington: The Science and Practice of Pharmacy,* cited *supra,* which describes materials and methods for preparing encapsulated pharmaceuticals.

Oral dosage forms, whether tablets, capsules, caplets, or particulates, may, if desired, be formulated so as to provide for gradual, sustained release of the active agent over an extended time period. Generally, as will be appreciated by those of ordinary skill in the art, sustained release dosage forms are formulated by dispersing the active agent within a matrix of a gradually hydrolyzable material such as a hydrophilic polymer, or by coating a solid, drug-containing dosage form with such a material. Hydrophilic polymers useful for providing a sustained release coating or matrix include, by way of example: cellulosic polymers such as hydroxypropyl cellulose, hydroxyethyl cellulose, hydroxypropyl methyl cellulose, methyl cellulose, ethyl cellulose, cellulose acetate, and carboxymethylcellulose sodium; acrylic acid polymers and copolymers, preferably formed from acrylic acid, methacrylic acid, acrylic acid alkyl esters, methacrylic acid alkyl esters, and the like, e.g. copolymers of acrylic acid, methacrylic acid, methyl acrylate, ethyl acrylate, methyl methacrylate and/or ethyl methacrylate; and vinyl polymers and copolymers such as polyvinyl pyrrolidone, polyvinyl acetate, and ethylene-vinyl acetate copolymer.

Preparations according to this invention for parenteral administration include sterile aqueous and nonaqueous solutions, suspensions, and emulsions. Injectable aqueous solutions contain the active agent in water-soluble form. Examples of nonaqueous solvents or vehicles include fatty oils, such as olive oil and corn oil, synthetic fatty acid esters, such as ethyl oleate or triglycerides, low molecular weight alcohols such as propylene glycol, synthetic hydrophilic polymers such as polyethylene glycol, liposomes, and the like. Parenteral formulations may also contain adjuvants such as solubilizers, preservatives, wetting agents, emulsifiers, dispersants, and stabilizers, and aqueous suspensions may contain substances that increase the viscosity of the suspension, such as sodium carboxymethyl cellulose, sorbitol, and dextran. Injectable formulations are rendered sterile by incorporation of a sterilizing agent, filtration through a bacteria-retaining filter, irradiation, or heat. They can also be manufactured using a sterile injectable medium. The active agent may also be in dried, e.g., lyophilized, form that may be rehydrated with a suitable vehicle immediately prior to administration via injection.

The compounds of the invention may also be administered through the skin using conventional transdermal drug delivery systems, wherein the active agent is contained within a laminated structure that serves as a drug delivery device to be affixed to the skin. In such a structure, the drug composition is contained in a layer, or "reservoir," underlying an upper backing layer. The laminated structure may contain a single reservoir, or it may contain multiple reservoirs. In one embodiment, the reservoir comprises a polymeric matrix of a pharmaceutically acceptable contact adhesive material that serves to affix the system to the skin during drug delivery. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form. Transdermal drug delivery systems may in addition contain a skin permeation enhancer.

In addition to the formulations described previously, the compounds may also be formulated as a depot preparation for controlled release of the active agent, preferably sustained release over an extended time period. These sustained release dosage forms are generally administered by implantation (e.g., subcutaneously or intramuscularly or by intramuscular injection).

Although the present compositions will generally be administered orally, parenterally, transdermally, or via an implanted depot, other modes of administration are suitable as well. For example, administration may be rectal or vaginal, preferably using a suppository that contains, in addition to the active agent, excipients such as a suppository wax. Formulations for nasal or sublingual administration are also prepared with standard excipients well known in the art. The pharmaceutical compositions of the invention may also be formulated for inhalation, e.g., as a solution in saline, as a dry powder, or as an aerosol.

It is to be understood that while the invention has been described in conjunction with the preferred specific embodiments thereof, the description above as well as the examples that follow are intended to illustrate and not limit the scope of the invention. Other aspects, advantages and modifications within the scope of the invention will be apparent to those skilled in the art to which the invention pertains.

### EXPERIMENTAL:

The practice of the present invention will employ, unless otherwise indicated, conventional techniques of synthetic organic chemistry, biological testing, and the like, which are within the skill of the art. Such techniques are explained fully in the literature. See, e.g., Fieser et al., Steroids (New York: Reinhold, 1959), Djerassi, Steroid Reactions: An Outline for Organic Chemists (San Francisco: Holden-Day, 1963), and Fried et al., Organic Reactions in Steroid Chemistry, vols. 1 and 2 (New York: Reinhold, 1972), for detailed information concerning steroid-related synthetic procedures. Reference may be had to Littlefield et al., Endocrinology 127: 2757-2762 (1990) and Wakeling et al., Endocrinology 99: 447-453 (1983) for a description of the biological testing procedures useful to evaluate compounds such as those described and claimed herein.

In the following examples, efforts have been made to ensure accuracy with respect to numbers used (e.g., amounts, temperature, etc.) but some experimental error and deviation should be accounted for. Unless indicated otherwise, temperature is in degrees C and pressure is at or near atmospheric. All reagents were obtained commercially unless otherwise indicated. ¹H NMR spectra were recorded on a Varian Gemini 300 MHz spectrometer or a similar instrument and are internally referenced. Data for ¹H NMR are reported as follows: chemical shift (δ ppm), multiplicity (s = singlet, d = doublet, t = triplet, q = quartet, m = multiplet), coupling constant (Hz), integration, and assignment.

### EXAMPLE 1

Preparation of (E)-3-Hydroxy-7α-methyl-21-[(2-dimethylamino)ethoxy]-19-norpregna-1,3,5(10),17(20)-tetraene (compound 2):

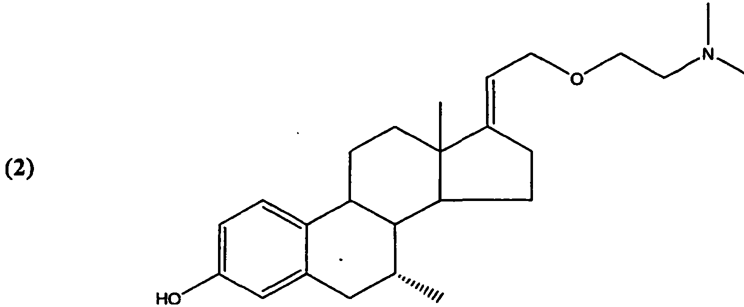

To a suspension of hexane-washed 60% NaH in mineral oil (2.5 g, 62.5 mmol) in anhydrous DMF (10 mL) at 0°C under argon was added 2-chloroethyldimethylamine hydrochloride (1.30 g, 9 mmol) in aliquots over 20 min and stirred until no further hydrogen was evolved. A solution of (E)-3-tetrahydropyranyloxy-7α-methyl-21-hydroxy-19-norpregna-1,3,5(10),17(20)-tetraene (1)

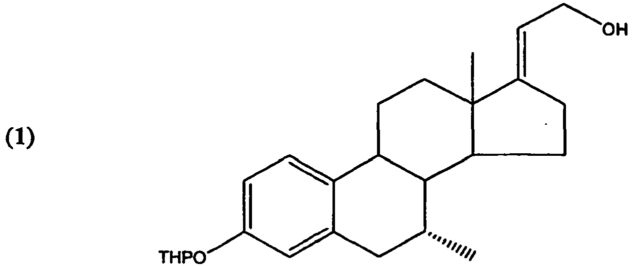

(synthesized as described in U.S. Patent No. 6,281,205 to Tanabe et al.) (1.2 g, 3.0 mmol) in 5 mL of DMF was added and the mixture stirred at room temperature for 10 min. n-Bu₄NI (0.11 g, 0.3 mmol) was added and the reaction heated to 70°C for 2.5 h, then cooled. The suspension was cautiously poured on to ice-/water and extracted with ethyl acetate. The combined extracts were washed with brine, dried over anhydrous MgSO₄, filtered, and concentrated to give 1.41 g (100%) of the tetrahydropyranyl (THP) ether of (2) as a gum.

To the crude THP ether (1.4 g) in 20 mL of MeOH was added at room temperature p-TsOH (0.6 g, 3.5 mmol) and the mixture stirred at room temperature for 3 h. The solution was poured on to saturated aqueous NaHCO₃ and extracted three times with EtOAc. The combined extracts were washed with brine, dried over anhydrous MgSO₄, filtered, and concentrated to give the crude solid product (**2**). Silica gel flash chromatography (eluted 1%, then 10% MeOH / CH₂Cl₂) yielded amine (**2**) as a white solid (0.586 g, 51%). ¹H NMR (300 MHz, CDCl₃) δ 0.79 (s, 3, CH₃), 0.82 (d, J = 7 Hz, 3, CH₃), 1.2-2.5 (m, 14), 2.42 (s, 6, N(CH₃)₂), 2.70 (m, 2), 3.05 (dd, J =17 Hz, J = 5 Hz, 1), 3.62 (m, 2), 3.99 (m, 2), 5.20 (m, 1, C=CH), 6.54 (d, J = 3 Hz, 1, ArH), 6.63 (dd, J = 8 Hz, J = 3 Hz, 1, ArH), 7.15 (d, J = 8 Hz, 1, ArH).

### EXAMPLE 2

Preparation of the citrate salt of (E)-Hydroxy-7α-methyl-21-[(2-dimethylamino)ethoxy]-19-norpregna-1,3,5(10),17(20)-tetraene:

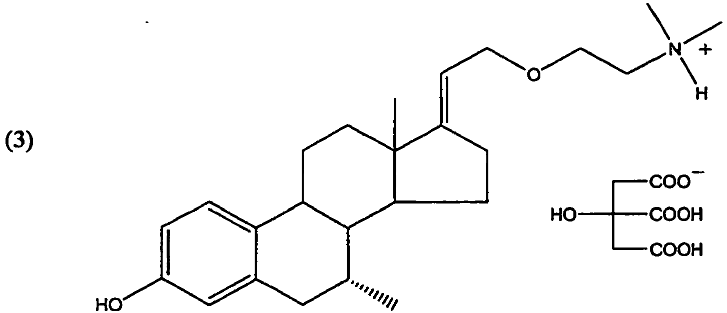

To a solution of the amine (**2**) (0.383 g, 1.0 mmol; synthesized as described in Example 1) in 30 mL of MeOH at room temperature was added citric acid (0.192 g, 1.0 mmol). The mixture was stirred for 15 min, then concentrated under reduced pressure and dried in vacuo. The citrate salt (**3**) (0.58 g, 100%) was obtained as a white solid. ¹H NMR (300MHz) (CD₃OD) δ 0.83 (d, J = 7 Hz, 3, CH₃), 0.84 (s, 3, CH₃), 1.3-2.5 (m, 14), 2.74 (d, J = 15 Hz, 2), 2.83 (d, J = 15 Hz, 2), 2.88 (s, 6, N(CH₃)₂), 3.00 (dd, J = 15 Hz, J = 5 Hz, 1), 3.32 (m, 2), 3.72 (m, 2), 4.07 (m, 2), 4.90 (broad s, OH), 5.24 (m, 1, C=CH), 6.47 (d, J = 2 Hz, 1, ArH), 6.54 (dd, J = 8 Hz, J = 2 Hz, 1, ArH), 7.09 (d, J = 8 Hz, 1, ArH).

### EXAMPLE 3

### EFFECTS OF 2 ON GROWTH OF HUMAN DERMAL MICROVASCULAR ENDOTHELIAL CELLS (HDMVEC)

To measure the inhibitory activity of (**2**) on growth of HDMVEC cells (Clonetics, San Diego, CA), 2,000 cells were seeded in each well of a 96-well plate in 200µl of EGM medium (Clonetics) supplemented with 5% fetal bovine serum (Sigma). The plate was incubated at 37 °C in a tissue culture incubator for 24 h, and then various concentrations of (2) dissolved in EGM were added to each well in 5-10 µl aliquots. Four wells were used for each concentration. Medium in each well was renewed with fresh test solution added every other day. After 7 days of culture, the viable cells were measured using reagents from an MTT kit (Promega Corporation, Madison, WI), using instructions provided by the supplier. The number of viable cells in treated wells relative to those in control wells gave the percentage of inhibition.

Results indicated a rapidly decreasing number of cells with increased dose of (2). The IC₅₀ value was about 80nM. This result demonstrates that (**2**) inhibited the growth of HDMVEC cells in a dose-response manner.

### EXAMPLE 4

### EFFECTS OF (2) ON ANTIANGIOGENIC ACTIVITY IN CHICK CHORIOALLANTOIC MEMBRANE (CAM) ASSAY

A CAM assay was conducted as previously described (K. Amin, J. Li, and W-R. Chao, Cancer Biol. Ther. 2: 173-178, (2003)). Briefly, fresh fertile eggs were incubated in a standard egg incubator at 37°C for 3 days. On Day 3, eggs were cracked under sterile conditions and embryos were placed into 20 × 100 mm sterile plastic Petri dishes and cultivated at 37°C in an embryo incubator with a water reservoir on the bottom shelf. Air was continuously bubbled into the water reservoir using a small pump so that the humidity in the incubator was kept constant. On Day 6, a sterile silicon "o" ring was placed on each CAM, and test compound (**2**) dissolved in 0.5% methylcellulose was delivered into each "o" ring under sterile conditions. Embryos were returned to the incubator. Control embryos received 10 µL of vehicle alone, while 12.5 µg of (**2**) was delivered to test embryos. On Day 8, embryos were removed from the incubator and kept at room temperature while blood vessel density was determined under each "o" ring using an image capturing system at a magnification of 160x. Results demonstrated that (**2**) drastically decreased the blood vessel density after 7 days of treatment when compared with the vehicle control.

### EXAMPLE 5

### EFFECTS OF (2) ON WOUND HEALING AND MICROVESSEL DENSITY

An *in vivo* fibrin-Z chamber assay (K. Amin et al., Cancer Biol. Ther. 2:173-178, (2003)) was run to analyze wound healing and microvessel density. Plasminogen-free fibrinogen and thrombin were added to a sterile dual porous chamber through a port. To implant the chambers into rats, the animals were anesthetized with 35 mg/kg of sodium pentobarbital. Two incisions approximately 2 cm in length each were made on the dorsum, one over the mid vertebral and the other over the lower vertebral regions. Pockets were made in the subcutaneous fascia lateral to the incisions using blunt end scissors, and the chambers were placed deep into the pockets. The incision wounds were closed with an autoclip stapling device. Triple antibiotic ointment was applied to the incision area to prevent infection. At 24 h after the implantation, rats began to receive a single daily oral dose of (**2**) at 0, 3, or 30 mg/kg for 12 days. At the end of 12^{th} day, animals were sacrificed. The chambers were harvested, fixed in 10% formalin overnight, and embedded in paraffin for histological analysis.

To determine the effect of (**2**) on wound healing response, granulation tissue (GT) thickness was measured in microns on H&E-stained cross sections of fibrin Z-chambers using the Zeiss Axioskop II equipped with digital camera (Axiocam) and digital imaging software KS 300. To measure the effect of (**2**) on microvessel density, immunohistochemistry was performed on 4 µm thick paraffin-embedded sections. The sections were deparaffinized in Hemo-D, rehydrated in graded alcohols, subjected to endogenous peroxidase block in 3% H₂O₂, boiled in 10% citrate buffer for antigen retrieval, blocked with 5% donkey serum, and placed overnight at 4°C with primary GT monoclonal antibody at 1:20 dilution. This was followed by washing and incubation with biotinylated donkey anti-mouse secondary antibody (Jackson Labs 1:1000) at 37°C for 20 minutes followed by incubation for 25 minutes with avidin-biotin peroxidase complex (ABC Kit, Vector Labs). Finally, the sections were developed with diaminobenzidine tetrahydrochloride (DAB) chromogen and counterstained with hematoxylin. Five fibrin Z-chambers with GT thickness closest to the mean for that group were selected for MVD estimation. Images of three hot spots (x200 fields showing maximal vessel density) were captured from each section and the blood vessels counted using the Zeiss interactive digital imaging software KS300. A total of 15 high-power fields (HPFs, 40×) were counted for vessel density for each group.

Results showed a much lower level of granulation and much thinner tissue after treatment with (**2**) at 30 mg/kg. The control tissue was significantly thicker than the treated tissue. A dose of 3 mg/kg (**2**) resulted in a decrease in thickness of about 20%, while a decrease of more than 35% in tissue thickness resulted from treatment with 30 mg/kg drug. Compound (**2**) dramatically inhibited the formation of granulation tissue. Further, there was a 30% decrease in microvessel formation as compared to the control.

### EXAMPLE 6

### EFFECTS OF (2) ON CELL PROLIFERATION IN VARIOUS TYPES OF CANCER CELL LINES

Compound (**2**) was evaluated in several different human cancer cell lines for its effects on cell growth. The human cancer cell lines used (ATCC) were A549 lung cancer cells, U-87 brain cancer cells, SKOV-3 ovarian cancer cells, PC-3 non-androgen dependent prostate cancer cells, and MDA MB-231 estrogen-independent breast cancer cells. Human umbilical vein endothelial cells (HUVEC) and a mouse transformed fibroblast cell line (L929) were also included in the assay for comparison. The assays were conducted using the same procedure as described in Example 3 for human dermal microvascular endothelial cells. The media used to culture the cell lines (Sigma) were RPMI-1640, Eagle's minimum essential medium, McCoy's 5A medium, and MEM medium for A549 and PC-3 cells, U-87 and MDA MB-231 cells, SKOV-3 cells, and L929 cells respectively. The medium used for HUVEC cells was EGM (Clonetics) supplemented with 5% fetal bovine serum. All other media were supplemented with 10% of fetal bovine serum.

The results indicated that cell number decreased rapidly between 0.1 and 1 µM in all cell cultures except L929, the mouse transformed fibroblast cell line. Thus, (**2**) had inhibitory effect on cell growth in all the human cancer cell lines tested, while the effect on growth of L929 mouse fibroblast cells was much less, requiring a higher concentration of (2) to induce growth inhibition.

### EXAMPLE 7

### EFFECTS OF (2) ON APOPTOSIS IN DU-145 HUMAN ANDROGEN-INDEPENDENT PROSTATE CANCER CELLS

DU-145 cells (ATCC) were treated with 10 µM (**2**) for 24 hours. Apoptosis was measured by the terminal deoxynucleotidyl transferase mediated dUTP nick end labeling (TUNEL) assay. This assay was conducted using the Apoptosis Detection Kit (Promega, Madison, WI) following the instructions provided by the manufacturer. Briefly, DU-145 cells were incubated with (2) for 24, 48, or 72 hours. At the end of incubation, cells were fixed with freshly prepared 4% methanol-free paraformaldehyde in PBS, washed, and then permeabilized in 0.2% Triton X-100 in PBS. The cells on the slides were then incubated with Tdt enzyme for 1 h at 37 °C, stained with propidium iodide, and examined under a fluorescence microscope. Fragmented DNA was made visible with green fluorescence stain within the apoptotic cells.

The untreated cells had very few, if any, fragments of DNA. On the other hand, quite a few pieces of fragmented DNA were evident in the treated cells, as a result of apoptosis.

### EXAMPLE 8

### EFFECTS OF (2) ON CELL CYCLE

DU-145 cells were treated with 10 µM of (**2**) for 6, 24, 48, 72, or 96 h. At the end of each time period, cells were hypotonically lysed in DNA staining solution (0.5 mg/ml propidium iodide, 0.1 % sodium citrate, and 0.05% Triton X-100). The stained cells were analyzed by flow cytometry (FACS) using software provided by Phoenix Flow System.

Results indicated that about 30% more cells were arrested in phase G1 when treated with (**2**) as compared with the untreated control sample, allowing a significantly smaller number of cells to progress through S into G2 phase.

### EXAMPLE 9

### EFFECTS OF (2) ON IN VIVO TUMOR GROWTH IN A XENOGRAFT NUDE MOUSE MODEL

Compound (**2**) was evaluated using the xenograft nude Balb-C athymic mouse model for its in vivo anti-tumor activity against PC-3 human prostate tumors. The mice were obtained from Taconic Laboratories. The experiment was conducted by subcutaneously implanting PC-3 cells to the right flanks of BALB-C athymic nude mice (Taconic Laboratories) followed by observing tumor growth daily. There were 8 mice in the control and each treatment groups. When tumor volumes reached 70-100 mm³, drug delivery was initiated at 3 dose levels of (**2**). The compound was delivered orally as follows: 0 mg/kg to the control group, 10 mg/kg to one treatment group, and 30 mg/kg to a second treatment group, each once daily for 28 days; and 100 mg/kg to a third treatment group, once weekly. Tumor volumes were measured twice weekly and body weights once weekly.

Tumors in the control group grew at a steady rate, while tumors in the 10 mg/kg group, 30 mg/kg group, and 100 mg/kg group had markedly decreased growth rates. This demonstrates that (**2**) potently inhibited the tumor growth rate.

There was dense microvascularization in the control group, but only minimal microvascularization in the group receiving 30 mg/kg. Microvascularization was reduced about 50% in the treated group as compared to the control group.

### EXAMPLE 10

### EFFECTS OF (2) COMBINATION WITH PACLITAXEL ON TUMOR GROWTH IN A XENOGRAFT NUDE MOUSE MODEL

Mice were treated as in Example 7, except that one group received no oral (**2**), but instead received 7.5 mg/kg i.p. paclitaxel (Sigma) when the tumors reached 70-100 mm³, while another group received both (**2**) orally at 10 mg/kg and paclitaxel i.p. at 7.5 mg/kg.

Results showed that (**2**) at 10 mg/kg or paclitaxel at 7.5 mg/kg produced similar profiles of growth inhibition in the mice, as compared to control. On the other hand, mice receiving a combination of the two drugs had much greater inhibition of growth over time.

### EXAMPLE 11

### ESTROGENIC ACTIVITY OF (2) IN AN ISHIKAWA CELL-BASED ASSAY

Absence of estrogenic activity caused by a cancer treatment drug is beneficial, as estrogen is implicated in the initiation and/or progression of certain tumors. The estrogenic effects of compound (**2**) were therefore investigated as follows.

Ishikawa cell based assays were run in parallel on human endometrial Ishikawa cells treated with compound (**2**) or with 2-methoxyestradiol. 2-Methoxyestradiol is a compound reported in the literature as an anti-angiogenic agent. Cells were cultured in 96-well plates for 48 hours in estrogen- and phenol red-free medium (DMEM/Hamm's F12 medium) containing 5% fetal bovine serum stripped of endogenous estrogens with dextran coated charcoal (Sigma). Compound (**2**) or other test compounds were then added to the cells and culturing was continued for an additional 72 hours. To the control cells (no (2)), 10⁻⁹ M of 2-methoxyestradiol was added. At the end of 72 hours, alkaline phosphatase activity was measured by adding 5 mM p-nitrophenyl phosphate, 0.24 mM MgCl₂, and 1 M diethanolamine (pH 9.8) to the cells while the 96-well plates were kept on ice. The plates were then warmed to room temperature, and the yellow color from the production of p-nitrophenol was measured after 2-3 hours using an ELISA plate reader at a wavelength of 405 nm. The estrogenic activity was defined as the alkaline phosphatase activity stimulated by the test compounds alone as a percentage of that stimulated by estradiol at 10⁻⁹ M. Stimulation by estradiol at 10⁻⁹ M was taken as 100%.

Results provided that cells treated with 2-methoxyestradiol produced a large amount of estrogenic activity, while **(2)**-treated cells produced essentially no such activity. Thus (2) does not stimulate production of estrogenic activity in these cells.

### EXAMPLE 12

### EFFECTS OF (2) ON THE PHOSPHORYLATION OF SIGNAL TRANSDUCERS AND ACTIVATORS OF TRANSCRIPTION-3 (STAT-3) IN DU-145 PROSTATE CANCER CELLS

It has been demonstrated that blocking phosphorylation of Stat-3 induces apoptosis of tumor cells and reduces VEGF-induced HMVEC cell migration and tube formation (R. Dhir et al., Prostate, 51 :241-246, (2002); and L.B. Mora et al., Cancer Res., 62:6659-6666, (2002)). Stat-3 is, therefore, an important molecular target for developing anti-cancer agents. DU-145 cells were treated with 0 (control), 100, or 500 nM of **(2)** for 48 hours. Western blot analysis was performed using total and phospho-Stat-3 (pStat-3, Tyr705) antibodies from Cell Signal Technology, Inc.

Western blot analysis showed that treatment of DU-145 cells with 0.5 µM of Compound **(2)** for 48 h reduced the tyrosine phosphorylation of Stat-3 by 52%, indicating that **(2)** inhibited phosphorylation of Stat-3 in DU-145 prostate cancer cells.

### EXAMPLE 13

### EFFECTS OF DIFFERENT COMPOUNDS OF THE INVENTION ON GROWTH OF HUMAN DERMAL MICROVASCULAR ENDOTHELIAL CELLS (HDMVEC)

The procedures of Example 3 were carried out to evaluate the inhibitory effect of various compounds of the invention on the growth of HDMVEC cells (Clonetics, San Diego, CA). The results are set forth in the following table:

| **Compound** | **Inhibitory Effect (IC₅₀ Value)** |
|---|---|
| 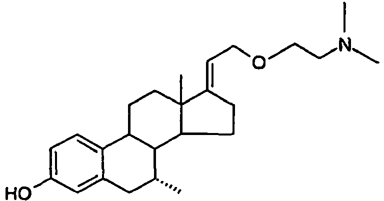 | 80 nM (0.08 µM) |
| 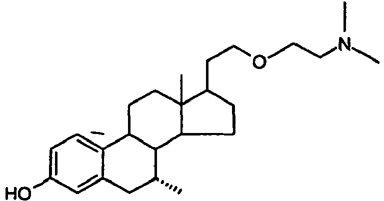 | 4 µM |
| 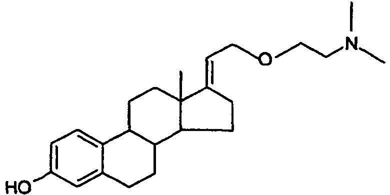 | 1.8 µM |

| | |
|---|---|
| 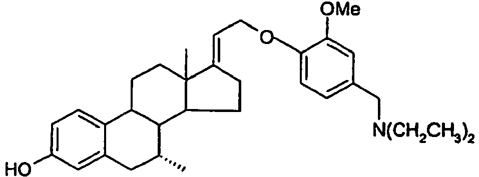 | 1.5 µM |
| 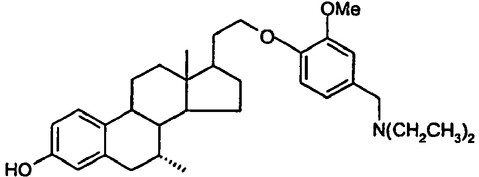 | 2 µM |
| 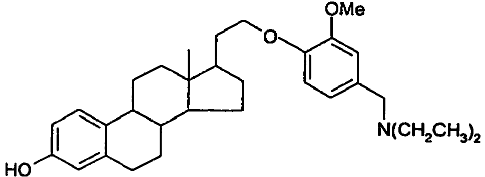 | 2 µM |
| 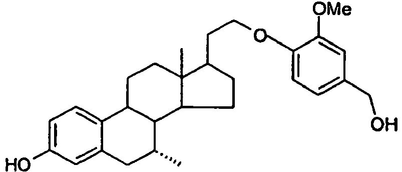 | 5.0 µM |
| 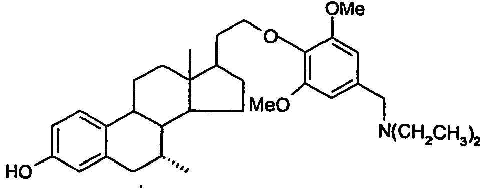 | 3.6 µM |

| | |
|---|---|
| 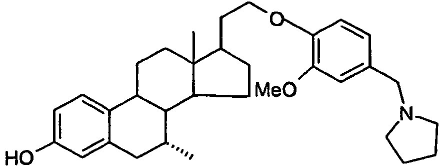 | 1.2 µM |
| 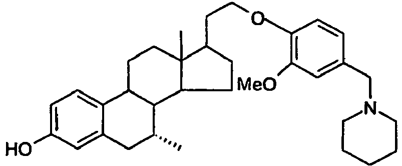 | 0.6 µM |
| 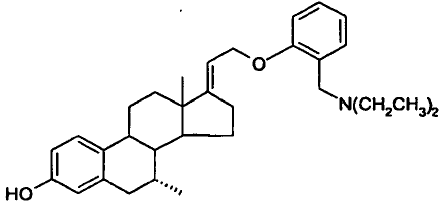 | 4.7 µM |
| 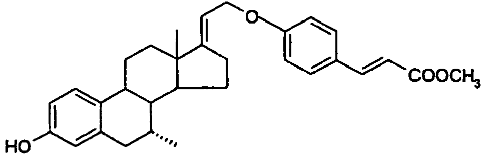 | 5.3 µM |
| 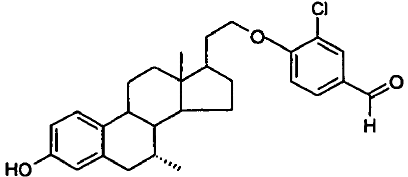 | 10 µM |

### EXAMPLE 14

### LOW OXYGEN (HYPOXIA) EXPERIMENTS

Hypoxia experiments were performed in either of two ways. To produce a very low ambient oxygen concentration (effectively anoxia), we used a protocol in which cells were incubated in a 5% CO₂-air atmosphere at 37 °C overnight and then placed in aluminum gas-exchange chambers maintained at 37 °C (Laderoute KR et al. Mol Cell Biol 22: 2515-2523, (2002); Laderoute KR et al. Mol Cell Biol 24: 4128-4137, (2004)). The chambers containing the cells were then placed in a 37°C circulating water bath and the original atmosphere was repeatedly exchanged with 5% CO₂-95% N₂ using a manifold equipped with a vacuum pump and a gas cylinder. Atmospheric oxygen partial pressure (pO₂) values of less than or equal to 0.01 % (relative to air at pO₂ of about 21 %) can be achieved inside the chambers using this system. Following various hypoxic exposures, the chambers were opened an anaerobic glove box (Bactron X, Sheldon Manufacturing Inc., Cornelius, OR; attached to a cylinder containing 5% CO₂-95% N₂) to prepare cell lysates without significant reoxygenation. All manipulations of hypoxic cells were performed in the anaerobic glove box. Atmospheric oxygen levels in both the chambers and the glove box have been measured and calibrated using a polarographic oxygen electrode (Oxygen Sensors, Inc., Norristown, PA). Hypoxia experiments involving a pO₂ of 1% were performed by equilibrating the glove box with an atmosphere of 1% O₂ 5% CO₂ 95% N₂ held at 37°C with a circulating fan. Cells were incubated inside a humidified space in the glove box and the medium on the cells was gently and continually agitated by using a rotary shaker. For these experiments, we used RAW 264.7 immortalized mouse macrophages as normal control cells, PC-3 prostate cancer cells, and MDA-MB-231 breast cancer cells, all available from ATCC.

We used immunoblotting to evaluate the experiments. Nuclear lysates were used to detect HIF-1α protein by immunoblotting (Murphy BJ et al. Biochem Biophys Res Commun (In Press): 2005). Briefly, cells were placed on ice in air or on Super Ice^{®} cold packs in the anaerobic glove box and the medium was removed. The cells were washed twice with deoxygenated, ice-cold PBS and then lysed by adding 800 µl of degassed, ice-cold lysis buffer 1 (LB1; 10 mM Tris-HCl, pH 8.0, 0.5% NP-40, 150 mM NaCl, 1 mM EDTA, 1X Protease Inhibitor Cocktail III, PIC III, Calbiochem). After spinning the lysates at 500 X g for 5 min at 4 °C, the supernatants were discarded and the pellets were resuspended in 500 µl of ice-cold LB2 (20 mM HEPES, pH 7.9, 400 mM NaCl, 1 mM EDTA, 1 mM DTT, 1 X PIC III). After spinning at 9,000 X g for 5 min at 4 °C, the protein concentrations of the supernatants were determined by using a bicinchoninic acid assay (Pierce Biotechnology). Equal protein samples (typically 5-10 µg) were resolved in 4-12% NuPage SDS-polyacrylamide gels (Invitrogen) and electroblotted onto Immobilon P membranes (Millipore). Blots were blocked in 5% nonfat dried milk in PBS containing 0.1 % Tween 20 at 4 °C overnight. For protein detection, blots were incubated for 1 h at room temperature with a primary antibody diluted in PBS-0.1% Tween 20 containing 5% nonfat dried milk, and a secondary anti-mouse or anti-goat IgG antibody conjugated with horseradish peroxidase (diluted 1:5,000). Primary antibody binding was detected and visualized by using the ECL Plus Western Blotting Detection System (Amersham Pharmacia Biotech) according to the supplier's instructions. HIF-1α protein was detected by using an anti-HIF-1α monoclonal antibody (Novus Biologicals, Cat. No. NB100-123; diluted 1:500) and a horseradish peroxidase-conjugated anti-mouse IgG secondary antibody (Santa Cruz Biotechnology, Cat. No. sc-2062; diluted 1:20,000). Detection of ERK1/2 protein was used both as a loading control and an internal standard for constitutive nuclear protein expression. ERK1/2 protein was detected by using an anti-mouse ERK1/2 antibody (Stressgen, Cat. No. KAP-MA001) and a horseradish peroxidase-conjugated goat anti-rabbit IgG secondary antibody (Santa Cruz Biotechnology, Cat. No. sc-2030).

We found that **(2)** had a potent inhibitory effect toward HIF-1α protein expression in PC-3 cells under anoxic, hypoxic, and even normoxic (5% CO₂-air; less than or equal to 5 µM, 6 h) conditions, without obvious cytotoxicity (i.e., the IC₅₀ for this inhibition is below that for cytotoxicity toward PC-3 cells).

We performed a similar study involving normoxic and anoxic MDA-MB-231 human breast carcinoma cells, and found that **(2)** was also a potent inhibitor of HIF-1α protein expression in these cells.

Results indicated that **(2)** is a novel inhibitor of HIF-1 activity in certain human carcinoma cell lines in which expression of the HIF-1α subunit is deregulated (and contributes to oncogenesis). In this connection, it is important therapeutically that **(2)** did not influence HIF-1α protein expression in RAW 264.7 macrophages (a "normal" cell line). HIF-1 activity is critical for the inflammatory response of the innate immune system. We did not find any effect of 2-methoxyestradiol (2-ME2; considered a standard HIF-1 inhibitor) on HIF-1α protein expression in either of these cancer cell lines under the same hypoxic/anoxic conditions as those used for our **(2)** studies. In addition, we did not find that YC-1, another standard HIF-1 inhibitor, could reproducibly inhibit HIF-1α protein expression under these conditions.

### EXAMPLE 15

### BIOLOGICAL PROFILE OF SR16388 FOR THE TREATMENT OF LUNG CANCER

**Compound (2) selectively binds to estrogen receptor-β with high affinity.** Human recombinant estrogen receptor-α (ER-α) and estrogen receptor-B (ER-β) were obtained from PanVera Corporation (Madison, WI) to conduct the binding assay. To measure the binding affinity of **(2),** various concentrations of **(2)** or 17β-estradiol were incubated with either ER-α or ER-β receptor in the presence of a fixed amount of ³H-17β-estradiol at 4 °C overnight. At the end of the incubation, the free- and bound-3H-estradiol were separated following the instructions provided by the manufacturer using hydroxylapatite. The result showed that the binding of **(2)** to ER-B was much higher than to ER-α with a binding affinity of 64 and 3 to ER-β and ER-α respectively (The binding affinity is defined as the amount of a compound that displaces 50% of ³H-estradiol relative to the amount of non-radiolabeled estradiol that displaces 50% of ³H-estradiol).

**Compound (2) potently inhibits the cell proliferation of A549 human non-small cell lung cancer cells (NSCLC).** To measure the inhibitory activity of test articles on growth of NSCLC cells, 2,000 cells were seeded in each well of a 96-well plate in 200µl of RPMI-1640 medium supplemented with 2 mM glutamine and 10% fetal bovine serum (growth medium). The plate was incubated at 37 °C in a tissue culture incubator for 24 h, and then various concentrations of the test articles dissolved in growth medium were added to each well in 5-10 µl aliquots. Four wells were used for each concentration. Medium in each well was renewed with fresh test solutions added every other day. After 7 days of culture, the viable cells were measured using reagents from MTT kit (Promega Corporation, Madison, WI), using instructions provided by the supplier. The number of viable cells in treated wells relative to those in control wells gave the percentage of inhibition. Compound **(2)** and Cisplatin were tested in parallel in the assay. Results showed that **(2)** inhibited the growth of NSCLC cells in a dose-response manner and the inhibitory activity was much more potent than that of Cisplatin.

Compound (2) alone or in combination with Cisplatin, Tarceva (Erlotinib), or Paclitaxel potently inhibited the growth of NSCLC tumors in a nude mouse xenograft model. The xenograft model was conducted using 8 weeks old female athymic nude mice supplied by Charles River Laboratories. After 4 days of quarantine, A549 human non small cell lung cancer cells (3 × 10⁶ cells/mouse) were subcutaneously implanted in the right flanks of mice in a 100 µl of Matrigel/phosphate buffered saline mixture (1:1). Animals were observed daily for tumor growth. When tumors became palpable, we began to measure the tumors using the formula V = W × L × H × π/6, where W and L represent the shorter and longer diameters of the tumor, H the height of the tumor. When tumor volumes reached approximately 50 mm³, animals were randomized into control and treatment groups of 10 mice each based on tumor volumes. Only the mice with tumor volumes closest to the mean value were used. On the day of randomization, animals began to receive drug treatment. Compound **(2)** and Tarceva (Erlotinib) were orally dosed via gavage once daily. Cisplatin and Paclitaxel were dosed via intraperitoneal injection once weekly.

After initiation of drug treatment, tumor volumes were measured twice weekly and body weights once weekly. The study was carried out for 30 days and unscheduled sacrifices were performed on animals with tumor volumes greater than 1500mm³ or tumors developing ulceration or loss of 20% of their original body weights. Animals were examined daily for adverse clinical signs related to the treatment received. At the end of 30 days, all mice were sacrificed. Throughout the entire study, no toxic effects were observed in the treatment groups. At the doses of 10 and 20 mg/kg, **(2)** drastically reduced the growth rate of A549 tumors. Compound **(2)** at 10 mg/kg in combination with Cisplatin at 3 mg/kg inhibited the tumor growth in a statistically significant manner (P<0.02) when compared with either drug alone at the same doses. The most impressive results were obtained when mice were treated with **(2)** in combination with Tarceva or Palitaxel. Compound **(2)** at 20 mg/kg in combination with Tarceva at 20 mg/kg or with Paclitaxel at 8 mg/kg completely blocked the tumor growth ((P< 0.02, 0.01, and 0.01 for **(2)** alone, Tarceva alone, or **(2)** + Tarceva respectively; P< 0.02 and 0.01 for SR16388 and **(2)** + Paclitaxel respectively).

## Claims

1. Use of a compound of formula (I) or (III) 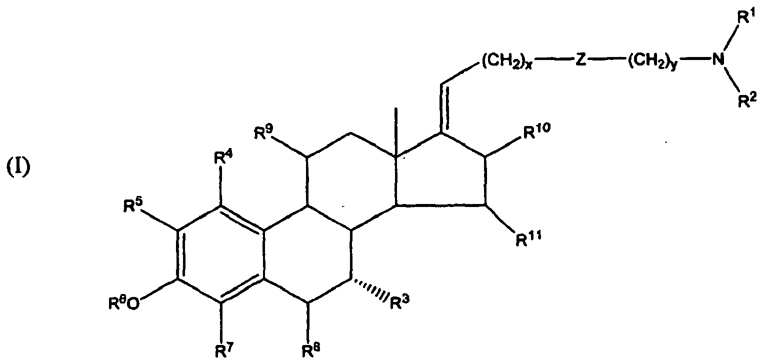 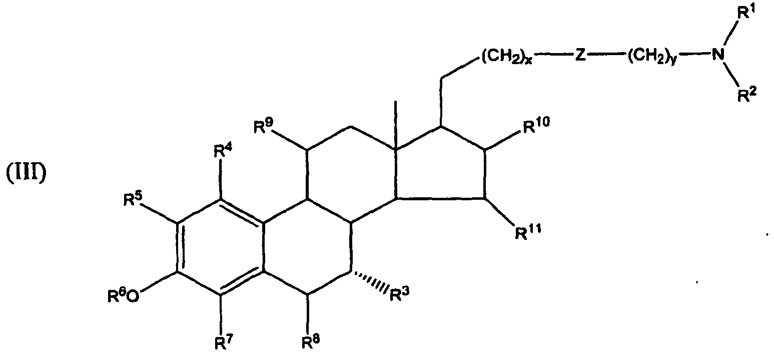 wherein:
Z is -O-(L)_{q}-, -S-(L)_{q}-, or -NR¹²-(L)_{q}- wherein q is zero or 1, L is monocyclic aryl optionally substituted with up to 4 substituents independently selected from hydrogen, hydroxyl, C₁-C₆ alkoxy, C₁-C₆ alkyl, halo, amino, and C₁-C₆ alkyl-substituted amino, wherein any two adjacent substituents on L may be taken together to form an optionally substituted cyclic structure, and R¹² is hydrogen or C₁-C₆ alkyl;
x is an integer in the range of 1 to about 6 inclusive;
when q is zero, y is an integer in the range of 2 to about 6 inclusive, and when q is 1, y is an integer in the range of 1 to about 6 inclusive;
R¹ and R² are independently selected from hydrogen and C₁-C₆ alkyl, or can be taken together to form an optionally substituted nitrogen heterocycle containing zero to two additional heteroatoms;
R³ is selected from hydrogen, hydroxyl, C₁-C₆ alkoxy, halo, C₁-C₃ alkyl, C₂-C₃ alkenyl, monocyclic aryl, and monocyclic aryl-substituted C₁-C₃ alkyl;
R⁴ is hydrogen or C₁-C₆ alkyl;
R⁵ is selected from hydrogen, C₁-C₆ alkoxy, halo, cyano, C₁-C₆ alkyl, and C₂-C₆ alkenyl;
R⁶ is selected from hydrogen, C₁-C₆ alkyl, C₂-C₁₂ acyl, and -SO₂NH₂;
R⁷ is selected from hydrogen, halo, C₁-C₆ alkyl, C₂-C₆ alkenyl, -OR¹³ and -SR¹³ where R¹³ is C₁-C₆ alkyl, C₂-C₆ acyl, or aryl;
R⁸ is hydrogen, C₁-C₆ alkoxy, or hydroxyl;
R⁹ is selected from hydrogen, hydroxyl, C₁-C₆ alkyl, C₂-C₆ alkenyl, aryl, and alkaryl; and
R¹⁰ and R¹¹ are independently selected from hydrogen, C₁-C₆ alkoxy, and C₁-C₆ alkyl,
or a pharmaceutically acceptable salt thereof for the preparation of a medicament for treating a disease or disorder involving angiogenesis or HIF-1 overexpression in a patient.

2. The use of claim 1, wherein R⁴, R⁵, R⁶, R⁷, R⁸, R⁹, R¹⁰, and R¹¹ are hydrogen, such that the compound has the structure of formula (II)

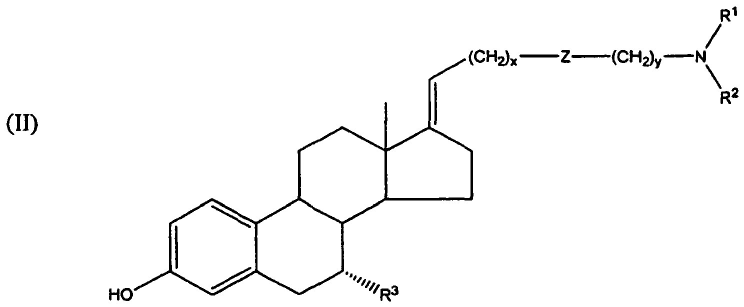

3. The use of claim 1, wherein R⁴, R⁵, R⁶, R⁷, R⁸, R⁹, R¹⁰, and R¹¹ are hydrogen, such that the compound has the structure of formula (IV)

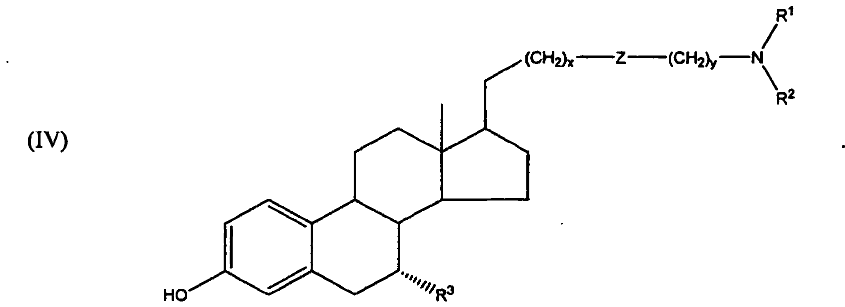

4. The use of any of claims 1 to 3, wherein x, y, Z, L, q, and R¹ through R¹³ are selected such that the molecular weight of the compound is at most about 750.

5. The use of any of claims 1 to 3, wherein x is 1 or 2 and y is 1 or 2.

6. The use of any of claims 1 to 3, wherein Z is O.

7. The use of any of claims 1 to 3, wherein R¹ is hydrogen, methyl, or ethyl, and R² is methyl or ethyl, or R¹ and R² are taken together to form a monocyclic nitrogen heterocycle.

8. The use of any of claims I to 3, wherein R³ is hydrogen or methyl.

9. The use of claim 2, wherein q is zero, such that Z is -O-, -S-, or -NR¹²-.

10. The use of claim 9, wherein x is 1, y is 2, R¹ and R² are methyl, R³ is methyl, and Z is -O-, such that the compound has the structure of formula **(2)**

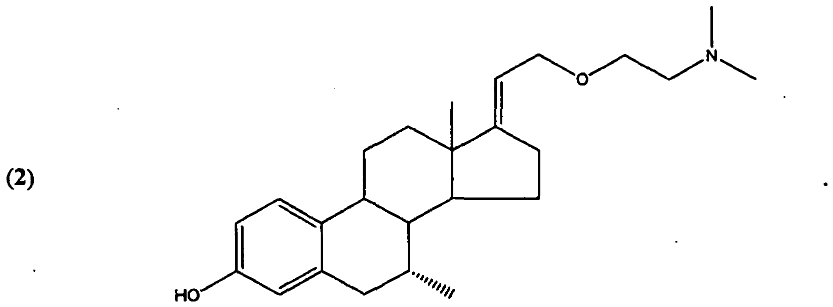

11. The use of claim 3, wherein q is 1 and L is phenyl or C₁-C₆ alkoxy-substituted phenyl.

12. The use of claim 3, wherein x is 1, y is 1, q is 1, L is 2-methoxyphenyl, R¹ and R² are ethyl, R³ is methyl, and Z is -O-, such that the compound has the structure of formula (V)

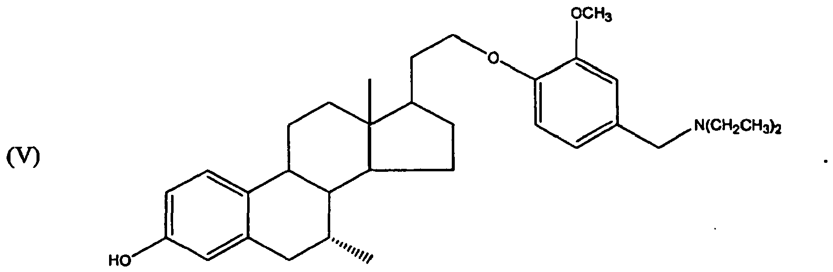

13. The use of any one of claims 1 to 3, 10 and 12, wherein the disease is a hormone-independent cancer, a chronic inflammatory disease, or detrimental neovascularization.

14. The use of claim 13, wherein the disease is a hormone-independent cancer selected from lung cancer and brain cancer.

15. The use of claim 13, wherein the disease is a chronic inflammatory disease selected from rheumatoid arthritis and psoriasis.

16. The use of any one of claims 1 to 3, 10 and 12, wherein the compound is administered in a pharmaceutical composition that additionally comprises a pharmaceutically acceptable carrier.

17. The use of claim 16, wherein the pharmaceutical composition is a unit dosage form.

18. The use of any one of claims 1 to 3, 10 and 12, wherein the compound is administered orally, parenterally, or transdermally.

19. The use of any one of claims 1 to 3, 10 and 12, further comprising co-administering a second active agent to the patient.

20. The use of claim 19, wherein the second active agent is an anticancer agent and the medical condition is cancer.

21. The use of a compound of claim 1 for the preparation of a medicament for inhibiting angiogenic activity, or HIF-1 overexpression, in mammalian tissue, comprising contacting the tissue with the compound.

22. The use of claim 21 for inhibiting the proliferation of mammalian endothelial cells, comprising contacting such cells with the compound.

23. The use of claim 21 further comprising an anticancer agent.

24. The use of claim 21 wherein the disease is prostate cancer.

25. The use of claim 21 for effecting cell cycle arrest of cancer cells in the G1 phase.

26. The use of claim 21 for inducing apoptosis in non-androgen dependent cancer cells.

27. The use of any one of claims 21 to 26, wherein R⁴, R⁵, R⁶, R⁷, R⁸, R⁹, R¹⁰, and R¹¹ are hydrogen, such that the compound has the structure of formula (II)

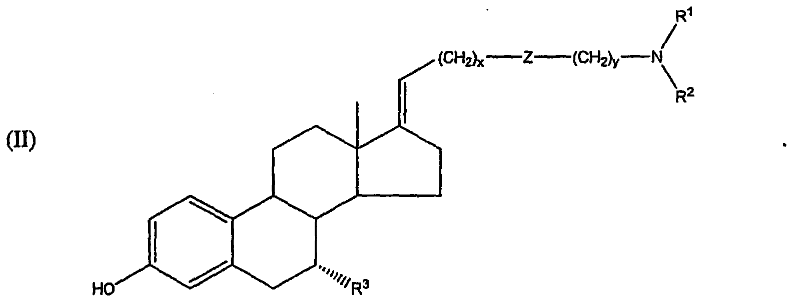

28. The use of any one of claims 21 to 26, wherein x, y, Z, L, q, and R¹ through R¹³ are selected such that the molecular weight of the compound is at most about 750.

29. The use of any one of claims 21 to 26, wherein x is 1 or 2 and y is 1 or 2.

30. The use of any one of claims 21 to 26, wherein Z is O.

31. The use of any one of claims 21 to 26, wherein R¹ is hydrogen, methyl, or ethyl, and R² is methyl or ethyl, or R¹ and R² are taken together to form a monocyclic nitrogen heterocycle.

32. The use of any one of claims 21 to 26, wherein R³ is hydrogen or methyl.

33. The use of claim 23, wherein the compound is administered orally.

34. The use of claim 33, wherein the anticancer agent is a taxane.

35. The use of claim 34, wherein the taxane is paclitaxel.

36. The use of claim 27, wherein q is zero, such that Z is -O-, -S-, or -NR¹²-.

37. The use of claim 36, wherein x is 1, y is 2, R¹ and R² are methyl, R³ is methyl, and Z is -O-, such that the compound has the structure of formula (2)

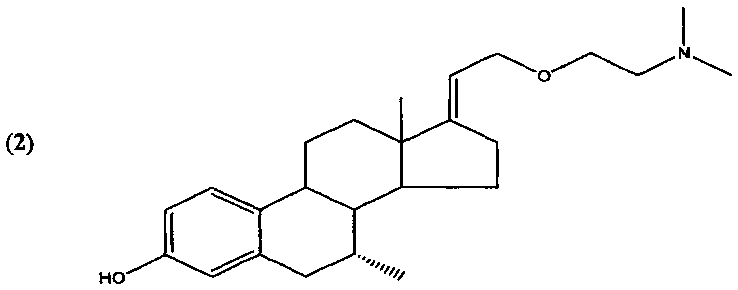

38. The use of claim 24, wherein the prostate cancer is non-androgen dependent.

39. An orally administrable pharmaceutical composition for treating a disease or disorder involving angiogenesis or HIF-1 overexpression in a patient comprising a carrier suitable for an oral dosage form and a therapeutically effective antiangiogenic or anti HIF-1 amount of a compound having the structure of formula (I) as defined in claim 1 or a pharmaceutically acceptable salt thereof.

40. A compound of formula (I) or (III) as defined in any one of claims 1 to 38, or a pharmaceutically acceptable salt thereof, for treating a disease or disorder involving angiogenesis or HIF-1 overexpression in a patient.

## Patentansprüche

1. Verwendung einer Verbindung der Formel (I) oder (III) 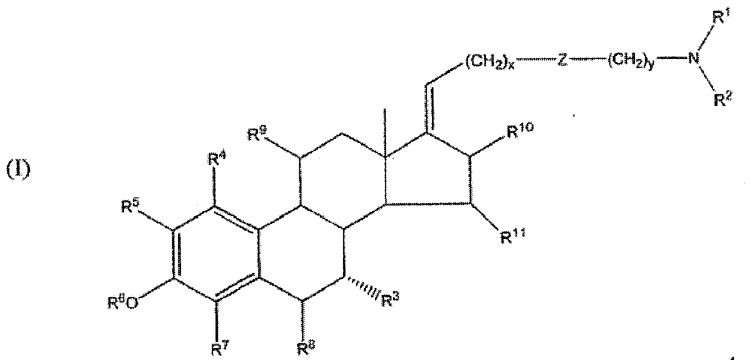 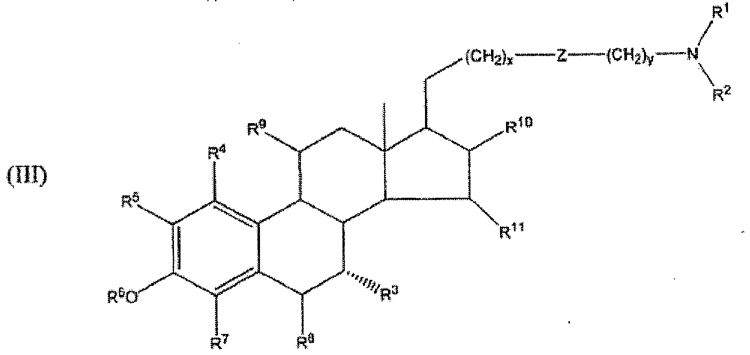 wobei gilt:
Z ist -O-(L)_{q}-, -S-(L)_{q}- oder -NR¹²-(L)_{q}-, wobei q null oder eins ist, L monozyklisches Aryl ist, das wahlweise mit bis zu 4 Substituenten ausgewählt aus Wasserstoff, Hydroxy, C₁-C₆-Alkoxy, C₁-C₆-Alkyl, Halogen, Amino und C₁-C₆-alkylsubstituiertes Amin substituiert ist, wobei zwei benachbarte Substituenten auf L zusammen eine wahlweise substituierte zyklische Struktur bilden können, und R¹² Wasserstoff oder C₁-C₆-Alkyl ist;
X ist eine ganze Zahl im Bereich von 1 bis etwa einschließlich 6,
wenn q null ist, ist y eine ganze Zahl im Bereich von 2 bis etwa einschließlich 6 und wenn q 1 ist, ist y eine ganze Zahl im Bereich von 1 bis etwa einschließlich 6;
R¹ und R² werden unabhängig ausgewählt aus Wasserstoff und C₁-C₆-Alkyl oder können zusammen einen wahlweise substituierten Stickstoffheterozyklus bilden, der null bis zwei weitere Heteroatome enthält;
R³ wird ausgewählt aus Wasserstoff, Hydroxy, C₁-C₆-Alkoxy, Halogen, C₁-C₃-Alkyl, C₂-C₃-Alkenyl, monozyklischem Aryl und monozyklischem Aryl-substituiertem C₁-C₃-Alkyl;
R⁴ ist Wasserstoff oder C₁-C₆-Alkyl;
R⁵ wird ausgewählt aus Wasserstoff, C₁-C₆-Alkoxy, Halogen, Cyano, C₁-C₆-Alkyl und C₂-C₆-Alkenyl;
R⁶ wird ausgewählt aus C₁-C₆-Alkyl, C₂-C₁₂-Acyl und SO₂NH₂;
R⁷ wird ausgewählt aus Wasserstoff, Halogen, C₁-C₆-Alkyl, C₂-C₆-Alkenyl, -OR¹³ und -SR¹³, wobei R¹³ C₁-C₆-Alkyl, C₂-C₆-Acyl oder Aryl ist;
R⁸ ist Wasserstoff, C₁-C₆-Alkoxy oder Hydroxy;
R⁹ wird ausgewählt aus Wasserstoff, Hydroxy, C₁-C₆-Alkyl, C₂-C₆-Alkenyl, Aryl und Alkaryl; und
R¹⁰ und R¹¹ werden unabhängig ausgewählt aus Wasserstoff C₁-C₆-Alkoxy und C₁-C₆-Alkyl,
oder ein pharmazeutisch annehmbares Salz davon zur Herstellung eines Arzneimittels zur Behandlung einer Erkrankung oder einer Störung, die mit Angiogenese oder HIF-1-Überexpression in einem Patienten verbunden ist.

2. Verwendung von Anspruch 1, wobei R⁴, R⁵, R⁶, R⁷, R⁸, R⁹, R¹⁰ und R¹¹ Wasserstoff sind, so dass die Verbindung die Struktur der Formel (II) aufweist.

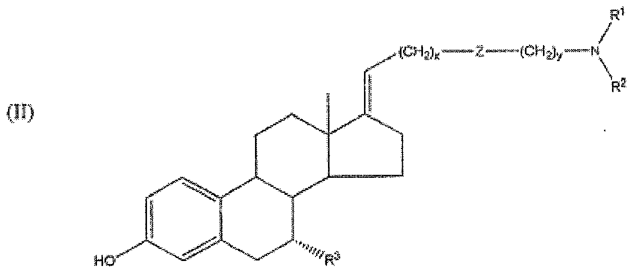

3. Verwendung von Anspruch 1, wobei R⁴, R⁵, R⁶, R⁷, R⁸, R⁹, R¹⁰ und R¹¹ Wasserstoff sind, so dass die Verbindung die Struktur der Formel (IV) aufweist.

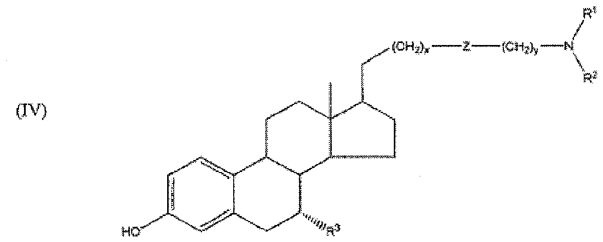

4. Verwendung von einem der Ansprüche 1 bis 3, wobei x, y, Z, L, q und R¹ bis R¹³ ausgewählt werden, so dass das Molekulargewicht der Verbindung höchstens etwa 750 beträgt.

5. Verwendung von einem der Ansprüche 1 bis 3, wobei x 1 oder 2 ist und y 1 oder 2 ist.

6. Verwendung von einem der Ansprüche 1 bis 3, wobei Z O ist.

7. Verwendung von einem der Ansprüche 1 bis 3, wobei R¹ Wasserstoff, Methyl oder Ethyl ist und R² Methyl oder Ethyl ist oder R¹ und R² zusammen einen monozyklischen Stickstoffheterozyklus bilden.

8. Verwendung von einem der Ansprüche 1 bis 3, wobei R³ Wasserstoff oder Methyl ist.

9. Verwendung von Anspruch 2, wobei q null ist, so dass Z - O-, -S- oder -NR¹² ist.

10. Verwendung von Anspruch 9, wobei x 1 ist, y 2 ist, R¹ und R² Methyl sind, R³ Methyl ist und Z -O- ist, so dass die Verbindung die Struktur der Formel (**2**) aufweist.

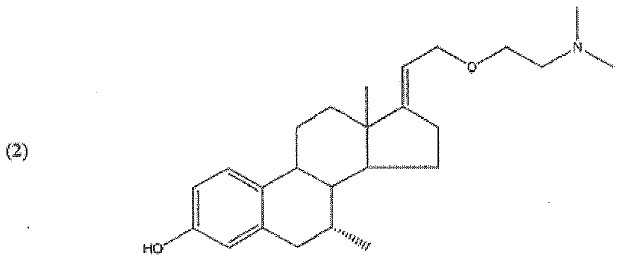

11. Verwendung von Anspruch 3, wobei q 1 ist, L Phenyl oder C₁-C₆-Alkoxy-substituiertes Phenyl ist.

12. Verwendung von Anspruch 3, wobei x 1 ist, y 1 ist, L 2-Methoxyphenyl ist, R¹ und R² Ethyl sind, R³ Methyl ist und Z - O- ist, so dass die Verbindung die Struktur der Formel (V) aufweist.

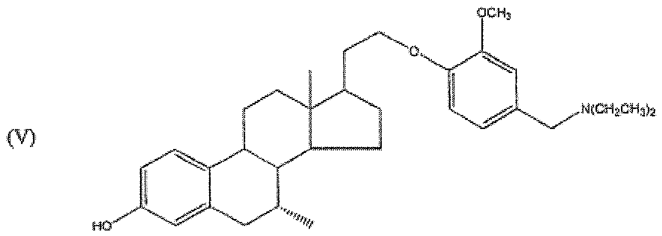

13. Verwendung von einem der Ansprüche 1 bis 3, 10 und 12, wobei die Erkrankung hormonunabhängiger Krebs, eine chronische Entzündungskrankheit oder unzuträgliche Neovaskularisierung ist.

14. Verwendung von Anspruch 13, wobei die Erkrankung hormonunabhängiger Krebs, ausgewählt aus Lungenkrebs und Gehirnkrebs, ist.

15. Verwendung von Anspruch 13, wobei die Erkrankung eine chronische Entzündungskrankheit, ausgewählt aus rheumatoider Arthritis und Psoriasis, ist.

16. Verwendung von einem der Ansprüche 1 bis 3, 10 und 12, wobei die Verbindung in einer pharmazeutischen Zusammensetzung verabreicht wird, die weiterhin einen pharmazeutisch annehmbaren Träger umfasst.

17. Verwendung von Anspruch 16, wobei die pharmazeutische Zusammensetzung eine Einheitsdosisform ist.

18. Verwendung von einem der Ansprüche 1 bis 3, 10 und 12, wobei die Verbindung oral, parenteral oder transdermal verabreicht wird.

19. Verwendung von einem der Ansprüche 1 bis 3, 10 und 12, weiterhin umfassend das dem Patienten Verabreichen eines zweiten Wirkstoffs als Begleitmedikation.

20. Verwendung von Anspruch 19, wobei der zweite Wirkstoff ein Krebsmittel ist und das medizinische Leiden Krebs ist.

21. Verwendung von Anspruch 1 zur Herstellung eines Arzneimittels zum Hemmen angiogener Aktivität oder HIF-1-Überexpression in Säugergewebe, umfassend das Kontaktieren des Gewebes mit der Verbindung.

22. Verwendung von Anspruch 1 zur Herstellung eines Arzneimittels zum Hemmen der Proliferation von Endothelialzellen von Säugern, umfassend das Kontaktieren dieser Zellen mit der Verbindung.

23. Verwendung von Anspruch 1 zur Herstellung eines Arzneimittels, weiterhin umfassend ein Krebsmittel.

24. Verwendung von Anspruch 1 zur Herstellung eines Arzneimittels zur Behandlung eines an Prostatakrebs leidenden Patienten.

25. Verwendung von Anspruch 1 zur Herstellung eines Arzneimittels zum Bewirken eines Zellzyklusarrests bei Krebszellen in der G1-Phase.

26. Verwendung von Anspruch 1 zur Herstellung eines Arzneimittels zum Induzieren der Apoptose in nicht androgenabhängigen Krebszellen.

27. Verwendung von einem der Ansprüche 21 bis 26, wobei R⁴, R⁵, R⁶, R⁷, R⁸, R⁹, R¹⁰ und R¹¹ Wasserstoff sind, so dass die Verbindung die Struktur der Formel (II) aufweist.

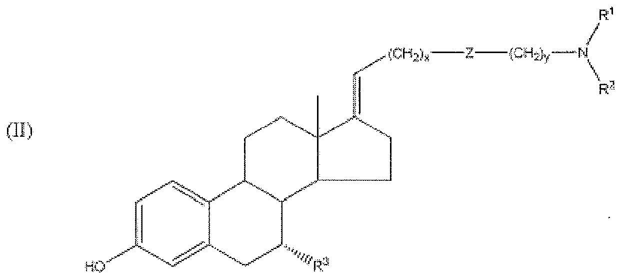

28. Verwendung von einem der Ansprüche 21 bis 26, wobei x, y, Z, L, q und R¹ bis R¹³ ausgewählt werden, so dass das Molekulargewicht der Verbindung höchstens etwa 750 beträgt.

29. Verwendung von einem der Ansprüche 21 bis 26, wobei x 1 oder 2 ist und y 1 oder 2 ist.

30. Verwendung von einem der Ansprüche 21 bis 26, wobei Z O ist.

31. Verwendung von einem der Ansprüche 21 bis 26, wobei R¹ Wasserstoff, Methyl oder Ethyl ist und R² Methyl oder Ethyl ist oder R¹ und R² zusammen einen monozyklischen Stickstoffheterozyklus bilden.

32. Verwendung von einem der Ansprüche 21 bis 26, wobei R³ Wasserstoff oder Methyl ist.

33. Verwendung von Anspruch 23, wobei die Verbindung oral verabreicht wird.

34. Verwendung von Anspruch 33, wobei das Krebsmittel ein Taxan ist.

35. Verwendung von Anspruch 34, wobei das Taxan Paclitaxel ist.

36. Verwendung von Anspruch 27, wobei q 0 ist, so dass Z - O-, -S- oder -NR¹²- ist.

37. Verwendung von Anspruch 36, wobei x 1 ist, y 2 ist, R¹ und R² Methyl sind, R³ Methyl ist und Z -O- ist, so dass die Verbindung die Struktur der Formel (2) aufweist.

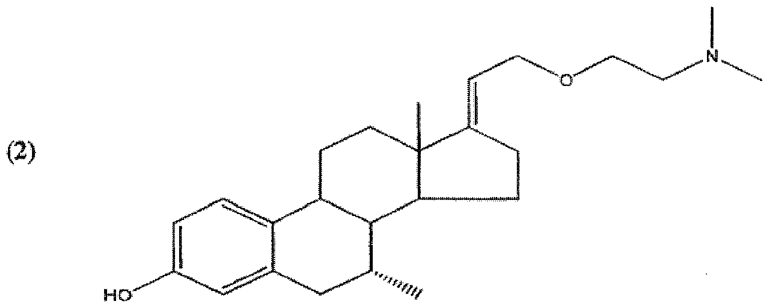

38. Verwendung von Anspruch 24, wobei der Prostatakrebs nicht androgenabhängig ist.

39. Oral verabreichbare pharmazeutische Zusammensetzung zur Behandlung einer Erkrankung oder einer Störung, die mit Angiogenese oder HIF-1-Überexpression in einem Patienten verbunden ist, umfassend einen Träger, der für eine orale Darreichungsform geeignet ist, und eine therapeutisch wirksame antiangiogene oder anti-HIF-1 Menge einer Verbindung, die eine Struktur der Formel (I), wie in Anspruch 1 definiert, aufweist, oder ein pharmazeutisch annehmbares Salz davon.

40. Verbindung der Formel (I) oder (III), wie in einem der Ansprüche 1 bis 38 definiert, oder ein pharmazeutisch annehmbares Salz davon, zur Behandlung einer Erkrankung oder einer Störung, die mit Angiogenese oder HIF-1-Überexpression in einem Patienten verbunden ist.

## Revendications

1. Utilisation d'un composé de formule (I) ou (III) : 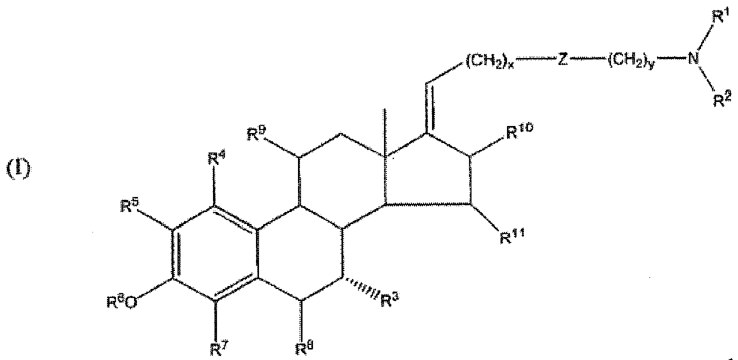 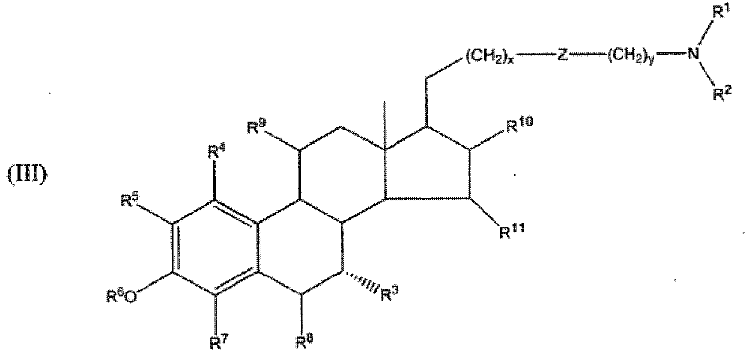 dans lesquelles
Z représente -O-(L)_{q}-, -S-(L)_{q}- ou -NR¹²-(L)_{q}- dans lesquels q vaut zéro ou 1, L représente un groupe aryle monocyclique éventuellement substitué par jusqu'à 4 substituants indépendamment choisis parmi un atome d'hydrogène, les groupes hydroxyle, alcoxy en C₁₋₆, alkyle en C₁₋₆, halogéno, amino et amino substitué un groupe par alkyle en C₁₋₆, dans lesquels deux substituants adjacents quelconques sur L peuvent être associés pour former une structure cyclique éventuellement substituée, et R¹² représente un atome d'hydrogène ou un groupe alkyle en C₁₋₆ ;
x est un nombre entier compris dans l'intervalle de 1 à environ 6, inclus ;
lorsque q vaut zéro, y est un nombre entier compris dans l'intervalle de 2 à environ 6, inclus, et lorsque q vaut 1, y est un nombre entier compris dans l'intervalle de 1 à environ 6, inclus ;
R₁ et R₂ sont indépendamment choisis parmi un atome d'hydrogène et les groupes alkyle en C₁₋₆, ou peuvent être associés pour former un hétérocycle azoté éventuellement substitué contenant de zéro à deux autres hétéroatomes ;
R³ est choisi parmi un atome d'hydrogène, les groupes hydroxyle, alcoxy en C₁₋₆, halogène, alkyle en C₁₋₃, alcényle en C₂₋₃, aryle monocyclique et alkyle en C₁₋₃ substitué par un groupe aryle monocyclique ;
R⁴ représente un atome d'hydrogène ou un groupe alkyle en C₁₋₆ ;
R⁵ est choisi parmi un atome d'hydrogène, les groupes alcoxy en C₁₋₆, halogène, cyano, alkyle en C₁₋₆ et alcényle en C₂₋₆ ;
R⁶ est choisi parmi un atome d'hydrogène, les groupes alkyle en C₁₋₆, acyle en C₂₋₁₂ et -SO₂NH₂ ;
R⁷ est choisi parmi les atomes d'hydrogène et d'halogène, les groupes alkyle en C₁₋₆, alcényle en C₂₋₆, -OR¹³ et -SR¹³ dans lesquels R¹³ représente un groupe alkyle en C₁₋₆, acyle en C₂₋₆, ou aryle ;
R⁸ représente un atome d'hydrogène, un groupe alcoxy en C₁₋₆ ou hydroxyle ;
R⁹ est choisi parmi un atome d'hydrogène, les groupes hydroxyle, alkyle en C₁₋₆, alcényle en C₂₋₆, aryle et alcaryle ; et
R¹⁰ et R¹¹ sont indépendamment choisis parmi un atome d'hydrogène, les groupes alcoxy en C₁₋₆ et alkyle en C₁₋₆,
ou d'un de leurs sels pharmaceutiquement acceptables, pour la préparation d'un médicament destiné au traitement d'une maladie ou d'un trouble impliquant une angiogenèse ou la surexpression de HIF-1 chez un patient.

2. Utilisation de la revendication 1, dans laquelle R⁴, R⁵, R⁶, R⁷, R⁸, R⁹, R¹⁰ et R¹¹ représentent des atomes d'hydrogène, de telle sorte que le composé présente la structure de formule (II)

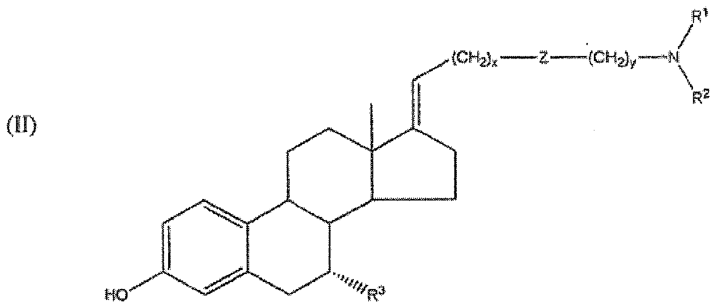

3. Utilisation de la revendication 1, dans laquelle R⁴, R⁵, R⁶, R⁷, R⁸, R⁹, R¹⁰ et R¹¹ représentent des atomes d'hydrogène, de telle sorte que le composé présente la structure de formule (IV)

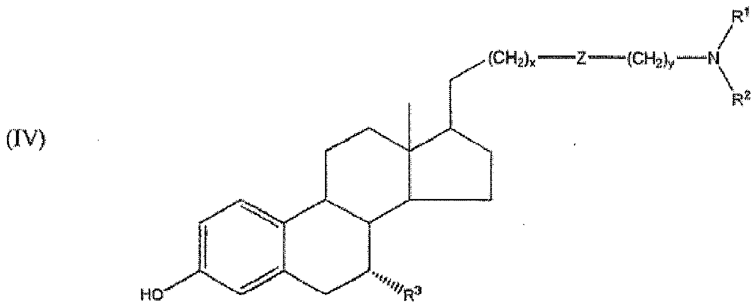

4. Utilisation de l'une quelconque des revendications 1 à 3, dans laquelle x, y Z, L, q et R¹ à R¹³ sont choisis de telle sorte que le poids moléculaire du composé soit au plus égal à environ 750.

5. Utilisation de la revendication 1 à 3, dans laquelle x vaut 1 ou 2 et y vaut 1 ou 2.

6. Utilisation de l'une quelconque des revendications 1 à 3, dans laquelle Z représente O.

7. Utilisation de l'une quelconque des revendications 1 à 3, dans laquelle R¹ représente un atome d'hydrogène, un groupe méthyle ou éthyle, et R² représente un groupe méthyle ou éthyle, ou R¹et R² sont associés pour former un hétérocycle azoté monocyclique.

8. Utilisation de l'une quelconque des revendications 1 à 3, dans laquelle R³ représente un atome d'hydrogène ou un groupe méthyle.

9. Utilisation de la revendication 2, dans laquelle q vaut zéro, de telle sorte que Z représente -O-, -S- ou -NR¹²-.

10. Utilisation de la revendication 9, dans laquelle x vaut 1, y vaut 2, R¹ et R² représentent un groupe méthyle, R³ représente un groupe méthyle et Z représente -O-, de telle sorte que le composé présente la structure de formule (2)

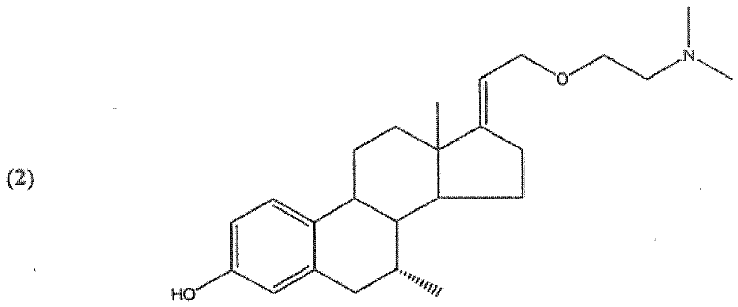

11. Utilisation de la revendication 3, dans laquelle q vaut 1 et L représente un groupe phényle ou phényle substitué par un groupe alcoxy en C₁₋₆.

12. Utilisation de la revendication 3, dans laquelle x vaut 1, y vaut 1, L représente un groupe 2-méthoxyphényle, R¹ et R² représentent un groupe éthyle, R³ représente un groupe méthyle et Z représente -O-, de telle sorte que le composé présente la structure de formule (V)

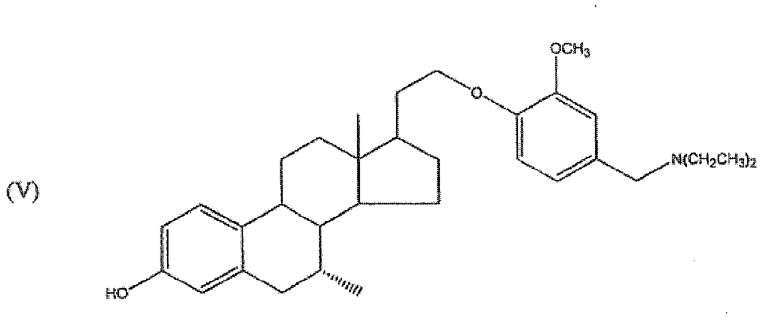

13. Utilisation de l'une quelconque des revendications 1 à 3, 10 et 12, dans laquelle la maladie est un cancer hormono-indépendant, une maladie inflammatoire chronique ou une néovascularisation nocive.

14. Utilisation de la revendication 13, dans laquelle la maladie est un cancer hormono-indépendant choisi parmi un cancer du poumon et un cancer du cerveau.

15. Utilisation de la revendication 13, dans laquelle la maladie est une maladie inflammatoire chronique choisie parmi la polyarthrite rhumatoïde et le psoriasis.

16. Utilisation de l'une quelconque des revendications 1 à 3, 10 et 12, dans laquelle le composé est administré dans une composition pharmaceutique qui comprend en outre un véhicule pharmaceutiquement acceptable.

17. Utilisation de la revendication 16, dans laquelle la composition pharmaceutique est une forme posologique unitaire.

18. Utilisation de l'une quelconque des revendications 1 à 3, 10 et 12, dans laquelle le composé est administré par voie orale, parentérale ou transdermique.

19. Utilisation de l'une quelconque des revendications 1 à 3, 10 et 12, comprenant en outre la co-administration d'un deuxième agent actif au patient.

20. Utilisation de la revendication 19, dans laquelle le deuxième agent actif est un agent anticancéreux et la condition médicale est un cancer.

21. Utilisation de la revendication 1 pour la préparation d'un médicament visant à inhiber l'activité angiogénique ou la surexpression de HIF-1 dans un tissu de mammifère, comprenant le fait de mettre le tissu en contact avec le composé.

22. Utilisation de la revendication 1 pour la préparation d'un médicament visant à inhiber la prolifération de cellules endothéliales de mammifère, comprenant le fait de mettre ces cellules en contact avec le composé.

23. Utilisation de la revendication 1 pour la préparation d'un médicament comprenant en outre un agent anticancéreux.

24. Utilisation de la revendication 1 pour la préparation d'un médicament destiné au traitement d'un patient souffrant d'un cancer de la prostate.

25. Utilisation de la revendication 1 pour la préparation d'un médicament visant à produire l'arrêt du cycle cellulaire des cellules cancéreuses en phase G1.

26. Utilisation de la revendication 1 pour la préparation d'un médicament visant à induire l'apoptose dans les cellules cancéreuses non androgéno-dépendantes.

27. Utilisation de l'un quelconque des revendications 21 à 26, dans laquelle R⁴, R⁵, R⁶, R⁷, R⁸, R⁹, R¹⁰ et R¹¹ représentent des atomes d'hydrogène, de telle sorte que le composé présente la structure de formule (II)

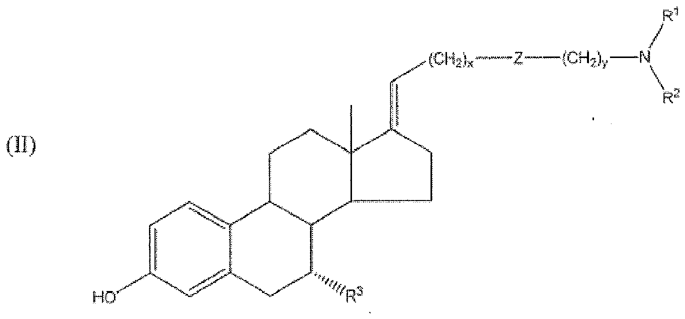

28. Utilisation de l'une quelconque des revendications 21 à 26, dans laquelle x, y Z, L, q et R¹ à R¹³ sont choisis de telle sorte que le poids moléculaire du composé soit au plus égal à environ 750.

29. Utilisation de l'une quelconque des revendications 21 à 26, dans laquelle x vaut 1 ou 2 et y vaut 1 ou 2.

30. Utilisation de l'une quelconque des revendications 21 à 26, dans laquelle Z représente O.

31. Utilisation de l'une quelconque des revendications 21 à 26, dans laquelle R¹ représente un atome d'hydrogène, un groupe méthyle ou éthyle, et R² représente un groupe méthyle ou éthyle, ou R¹ et R² sont associés pour former un hétérocycle azoté monocyclique.

32. Utilisation de l'une quelconque des revendications 21 à 26, dans laquelle R³ représente un atome d'hydrogène ou un groupe méthyle.

33. Utilisation de la revendication 23, dans laquelle le composé est administré par voie orale.

34. Utilisation de la revendication 33, dans laquel le l'agent anticancéreux est un taxane.

35. Utilisation de la revendication 34, dans laquelle le taxane est le paclitaxel.

36. Utilisation de la revendication 27, dans laquelle q vaut zéro, de telle sorte que Z représente -O-, -S- ou -NR¹²-.

37. Utilisation de la revendication 36, dans laquelle x vaut 1, y vaut 2, R¹ et R² représentent un groupe méthyle, R³ représente un groupe méthyle et Z représente -O-, de telle sorte que le composé présente la structure de formule (2)

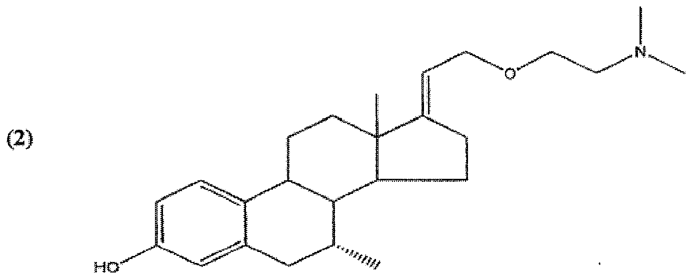

38. Utilisation de la revendication 24, dans laquelle le cancer de la prostate n'est pas androgéno-dépendant.

39. Composition pharmaceutique pouvant être administrée par voie orale, destinée au traitement d'une maladie ou d'une affection impliquant une angiogenèse ou la surexpression de HIF-1 chez un patient, comprenant un véhicule approprié à une forme posologique orale et une quantité antiangiogénique ou anti-HIF-1 thérapeutiquement efficace d'un composé présentant la structure de la formule (I) définie dans la revendication 1 ou d'un de ses sels pharmaceutiquement acceptables.

40. Composé de formule (I) ou (III) défini dans l'une quelconque des revendications 1 à 38, ou sel pharmaceutiquement acceptable de celui-ci, destiné au traitement d'une maladie ou d'une affection impliquant une angiogenèse ou la surexpression de HIF-1 chez un patient.
